# EUROPEAN PATENT APPLICATION

(11) **EP 4 275 701 A1**
(43) Date of publication of application: **15.11.2023**
(21) Application number: 22173383.5
(22) Date of filing: 13.05.2022
(51) Int. Cl.: A61K 39/395, A61P 35/00, A61P 37/00, C07K 16/28

(54) **CONDITIONING REGIMEN FOR CELL TRANSPLANT**

(71) Applicant: Hansa Biopharma AB, 220 07 Lund (SE)
(72) Inventor: The designation of the inventor has not yet been filed
(74) Representative: J A Kemp LLP

(57) **Abstract**

The present invention relates to a conditioning regimen for the transplant of a cell to a subject, optionally wherein the cell is a hematopoietic stem / progenitor cells (HSPC). The invention also relates to methods for the prevention or treatment of a disease or condition in a subject by administration of a cell transplant, wherein said administration comprises the conditioning regimen of the invention.

## Description

### Field of the Invention

The present invention relates to a conditioning regimen for the transplant of a cell, optionally hematopoietic stem / progenitor cells (HSPC), to a subject. The invention also relates to methods for the prevention or treatment of a disease or condition in a subject by administration of a cell transplant, wherein said administration comprises the conditioning regimen of the invention.

### Background of the Invention

The complex immunology involved in the transplant of cells, such as HSPC, can be problematic, particularly if there is sensitization to donor antigens prior to transplantation. The presence of donor and recipient immune systems can lead to acute and chronic rejection with both humeral and cellular components. Vigorous host versus graft reactions (HVG) and graft versus host disease (GVHD) are both observed. Often, the transplanted cells fail to successfully engraft in the recipient. Current methods seek to address these problems by pre- and post-transplant immunosuppression. Steps carried out pre-transplant may be referred to as a conditioning regimen and may include treatments that are not solely immunosuppressive. For example, radiation may be used to deplete some or all of the existing bone marrow cells in the recipient, creating space for engraftment of the transplanted cells. However, lymphocyte depleting agents are often used. Using high doses of these agents leads to a desired reduction of GvHD, but at the cost of delayed immune reconstitution or even increased host engraftment failure (because the agents also act on the transplanted cells), thereby enhancing the risk of severe infectious complications and reducing overall survival (OS). There is a need for improved conditioning regimens for the transplant of cells.

### Summary of the Invention

Conditioning regimens for the transplant of cells, such as HSPC, typically include a step of reducing the numbers and/or down-modulating the activity of lymphocytes (preferably T lymphocytes) in the subject prior to transplant. This may be referred to as lymphocyte depletion, or lymphodepletion. It may be achieved by administration of an agent comprising IgG molecules. Preferred examples include rabbit antithymocyte globulin (rATG) or an anti-CD52 monoclonal antibody (e.g. alemtuzumab, CAMPATH). Whilst a reduction in lymphocyte numbers and/or activity may be beneficial in terms of improved cell engraftment, it of course also has negative effects, in that the agents responsible may also attack the transplanted cells, and the patient will have a less effective immune system until the effects are reversed, e.g. by recovery of lymphocyte numbers.

The positive and negative aspects of reducing the numbers and/or down-modulating the activity of lymphocytes therefore require careful balance. For example, conditioning regimens may use a lower intensity lymphocyte depletion (e.g. lower / less frequent doses of cell depleting agent) so that immune recovery is faster after transplant is complete, but this may result in less effective transplant and/or a slower transplant procedure overall if a longer delay is required between initiation of lymphocyte depletion and the transplant. Alternatively conditioning regimens may use higher intensity lymphocyte depletion (e.g. higher / more frequent doses of cell depleting agent), but this may result in a longer time to immune recovery in the patient, and the depleting agent may remain in sufficient quantities to attack the transplanted cells.

The present inventors have surprisingly shown that a conditioning regimen including enzymatic inactivation of serum IgG in a subject results in significant benefits by making this balance easier to achieve, when used to rapidly inactivate an IgG-based agent used to reduce the numbers and/or down-modulate the activity of lymphocytes after it has had its positive effects, thereby reducing the time in which it may exert negative effects.

The present invention provides a conditioning regimen for the transplant of a cell to a subject, comprising:
a. administering to the subject an agent comprising IgG molecules, which agent reduces the numbers and/or down-modulates the activity of lymphocytes in the subj ect;
b. subsequently administering to the subject an enzyme which inactivates serum IgG molecules in the subject; and
c. subsequently administering the transplant to the subject.

The present invention also provides a method for the prevention or treatment of immune rejection of a cell transplant, the method comprising administering a cell transplant to a patient in accordance with the conditioning regimen of the invention.

The present invention also provides a method for the prevention or treatment of a disease or condition that is prevented or treated by cell transplant, the method comprising administering a cell transplant to a patient in accordance with the conditioning regime of the invention. The disease or condition may be selected from Hematological malignancies; Solid tumor cancers; Hematologic diseases; Anemias; Myeloproliferative disorders; Metabolic disorders; Environmentally-induced diseases; Viral diseases; Autoimmune diseases; Lysosomal storage disorders; and Immunodeficiencies.

The present invention also provides a method for the treatment of alemtuzumab overdose, comprising administering to a patient in need thereof a therapeutically effective amount of an enzyme which inactivates serum IgG molecules in the subject.

### Brief Description of the Figures

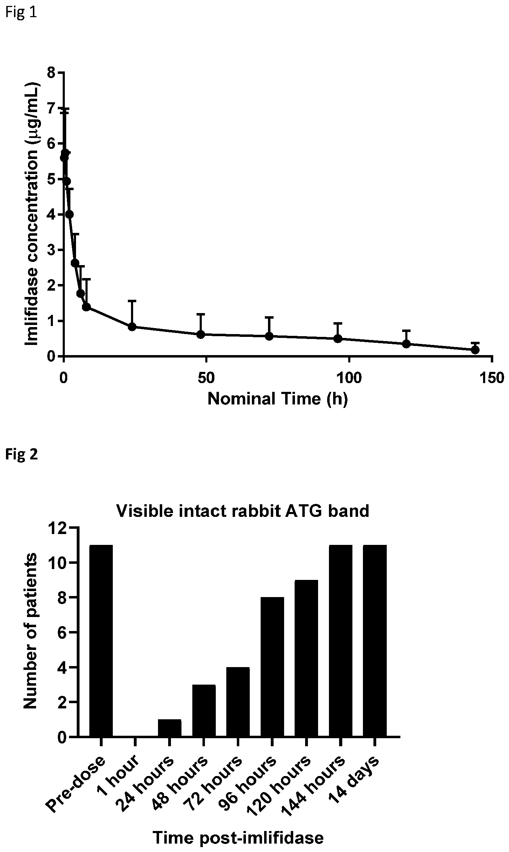

**Fig 1****. Mean imlifidase concentration vs. nominal time from dosing** (N=15). Data BLQ are included in mean calculation as BLQ/2. SD indicated with bars.
**Fig 2****. In vitro cleavage of rATG by imlifidase over time.** Columns indicate number of subjects with visible intact rATG on **Western** blot post-imlifidase (N=11).

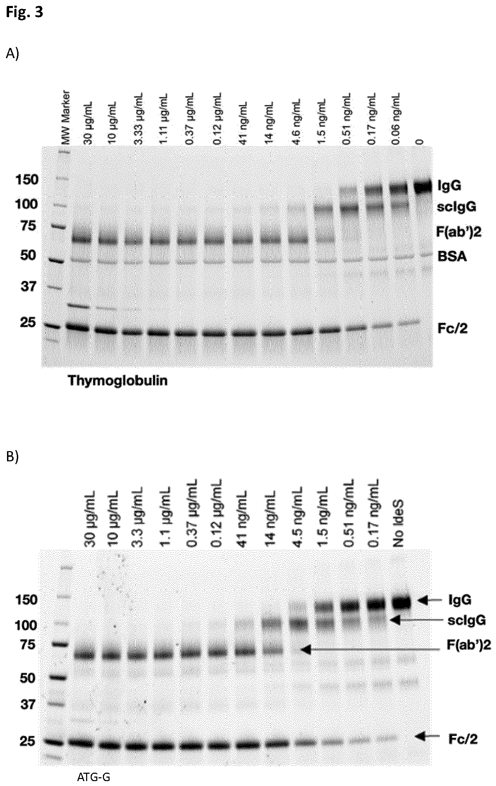

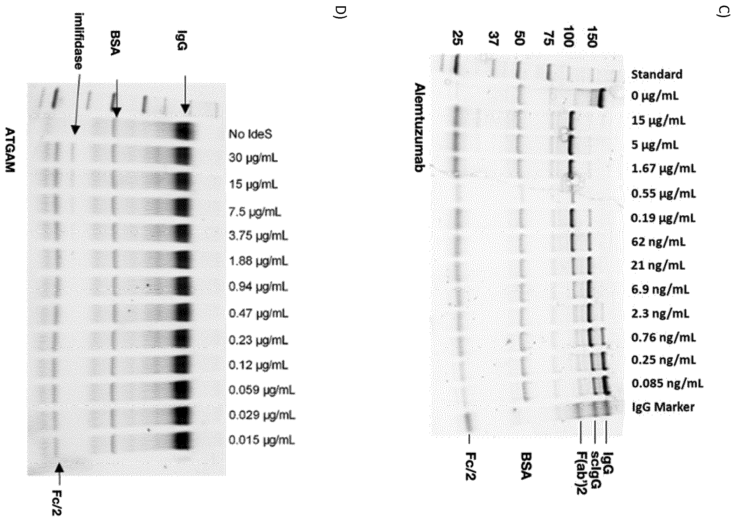

**Fig.3****. Imlifidase cleaves rabbit and human but not horse IgG-based therapeutic antibodies.** IgG-based biologics have been incubated with a titration of imlifidase supplemented with BSA as carrier protein. Samples were incubated for 90 min at 37°C before being separated on SDS-PAGE (4-20% gradient). Polyclonal rabbit IgG mixtures at 1mg/mL (panels A and B), and the human IgG1-based anti-CD52 mAb alemtuzumab at 1 mg/mL (panel C), are easily cleaved into single- and fully cleaved IgG by imlifidase. Equine ATGAM (1mg/mL) (panel D) is largely resistant to imlifidase cleavage, only a small fraction can be detected to be cleaved, as evidenced by the faint formation of Fc/2 when incubated with imlifidase.

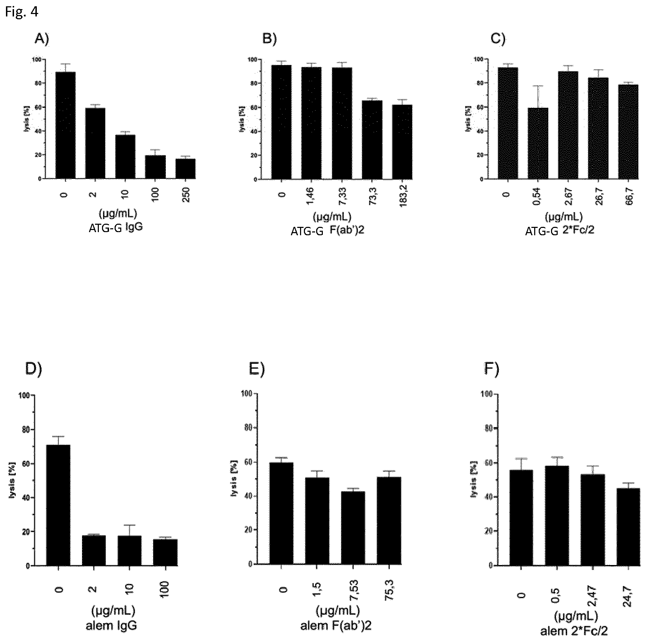

**Fig.4****. Imlifidase has an effect on NK cell survival after rATG and alemtuzumab challenge. Cytotoxicity of NK cells against K562 cells and the role of rATG and alemtuzumab measured with cytotoxicity calcein release assay.** Cytotoxic activity against casein-labeled K562 target cells was measured as a readout for survival and functionality of eNK cells after preincubation with intact rATG (Grafalon) (A) or intact alemtuzumab (D). Both reagents impact the capacity of eNK cells to efficiently lyse the K562 target cells. rATG shows a dose-dependent cytotoxicity in the tested range of 2 to 250 µg/mL. After incubation of eNK cells with 100 and 250 µg/mL rATG the least lysis (%) of the K562 target cells can be observed (A). Lysis of K562 target cells by eNK cells is already prevented at 2 µg/mL alemtuzumab (D). Challenging the eNK cells with rATG F(ab')2 preparations or with equimolar concentrations of the intact rATG protects the eNK effector cells, allowing only for an partially increased lysis of K562 target cells (B) even at 73,3 and 183,2 µg/mL F(ab')2. In contrast, alemtuzumab F(ab')2 fragments hardly have any cytotoxic effect on eNK cells, even allowing them to handle concentrations of alemtuzumab-F(ab')2 as high as 75,3 µg/mL (E). Imlifidase-generated 2^{∗}Fc/2 from rATG (C) and alemtuzumab 2^{∗}Fc/2 (F) do not seem to have a cytotoxic effect on eNK cells.

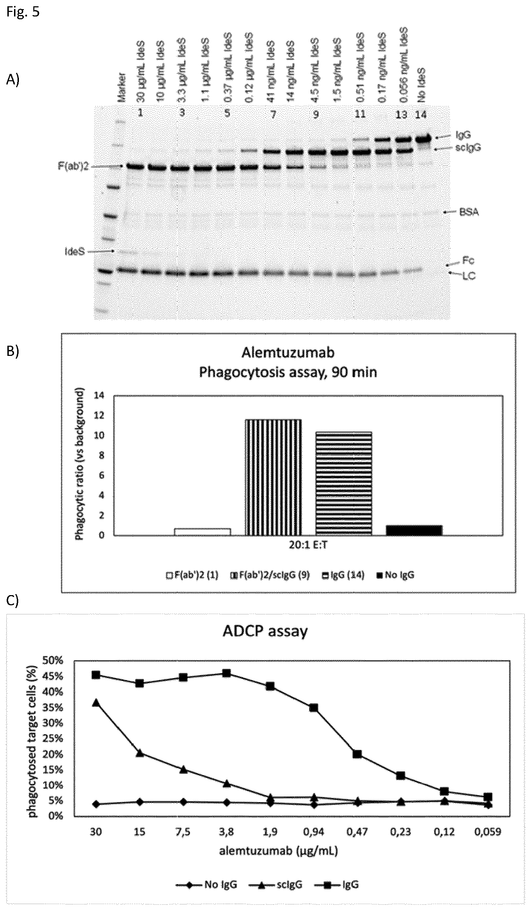

**Fig. 5** **ADCP effector function of alemtuzumab is prevented by imlifidase.** Alemtuzumab was pre-treated with different concentrations of imlifidase and separated on SDS PAGE (4-20%) to identify samples with intact IgG (well 14), F(ab')2 (well 1) and scIgG (well 9) fragments (A). Calcein-labeled NuDUL1 target cells were opsonized with 30 µg/mL of the respective alemtuzumab preparations before incubation with FarRed labeled THP1 effector cells. After 90 min at 37°C, the cells were analyzed by flow cytometry to identify double positive, i.e. phagocytosed cells. At a concentration of 30 µg/mL, scIgG has the same ability to induce phagocytosis as intact alemtuzumab. Fully cleaved F(ab')2 alemtuzumab does not opsonize the NuDUL1 cells for phagocytosis by THP1 cells (B). scIgG- and intact-alemtuzumab were further compared using different concentrations for opsonization. scIgG alemtuzumab loses its opsonization effect down to background levels at around 2 µg/mL while intact alemtuzumab still has the same activity as seen at 30 µg/mL (C).

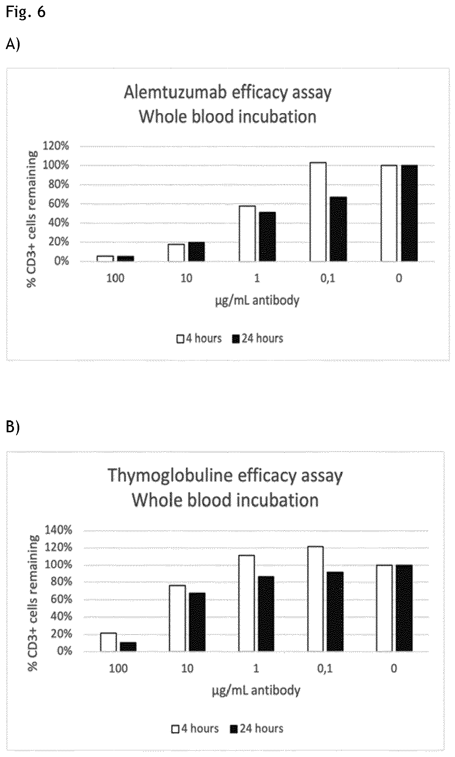

**Fig. 6** **CD3+ cells are depleted in whole blood *in vitro* by alemtuzumab and thymoglobulin (rATG) in dose dependent manner.** Peripheral blood was collected from a healthy volunteer in sodium heparin tubes. The whole blood was incubated with titrations of alemtuzumab (A) and thymoglobulin (ATG, Genzyme) (B) for 4 and 24 hours. After RBC lysis the cells were stained with anti-CD3-PE antibody and analyzed using a Accuri C6 flow cytometer.

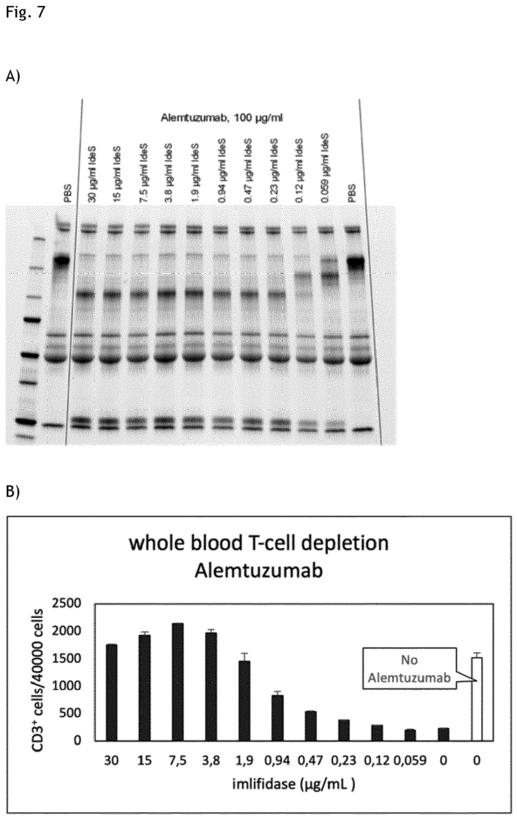

**Fig. 7** **Alemtuzumab-induced CD3+ T-cell depletion in whole blood can be prevented with imlifidase.** Peripheral blood was collected from a healthy volunteer in sodium heparin tubes and pre-treated with indicated amounts of imlifidase. After 6 hours alemtuzumab (100 µg/mL) was added and incubated for another 16 hours. An aliquot of the samples was removed for plasma preparation. The plasma was analyzed running an SDS-PAGE to follow the IgG cleavage in the blood (A). RBC were lysed from the remaining samples and the PBMC stained with CD3-PE for flow cytometry using an Accuri C6 to detect the T cells. The data shows that alemtuzumab can efficiently deplete the CD3-positive cell population in whole blood and that a concentration of approx. 4 µg/mL imlifidase is sufficient to cleave all serum IgG and 100 µg/mL alemtuzumab (B).

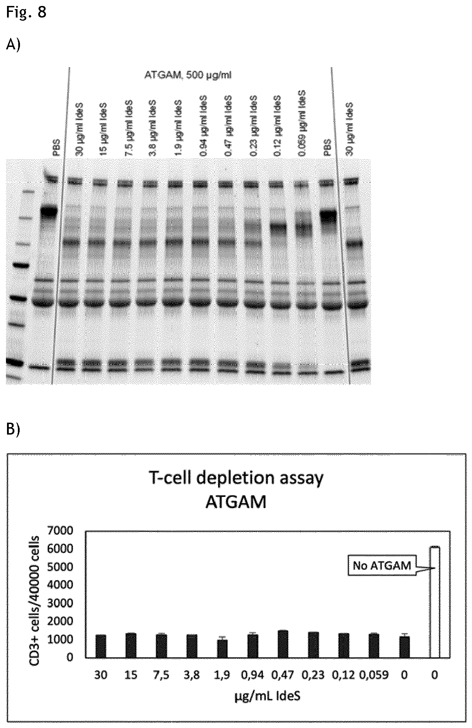

**Fig. 8****. Imlifidase treatment does not protect CD3+ T-cells from depletion by ATGAM.** Whole blood from a healthy donor was treated in vitro for 6 hours with indicated concentrations of imlifidase before adding 500 µg/mL ATGAM or horse IgG (no ATGAM) as control for 16 hours. Plasma aliquots were prepared for SDS-PAGE analysis. Precision Plus MW standard (5 µL) was used as control (A). The remaining samples were lysed for RBC and labelled with PE-conjugated anti-CD3 antibody and analyzed on Accuri C6 flow cytometer (B).

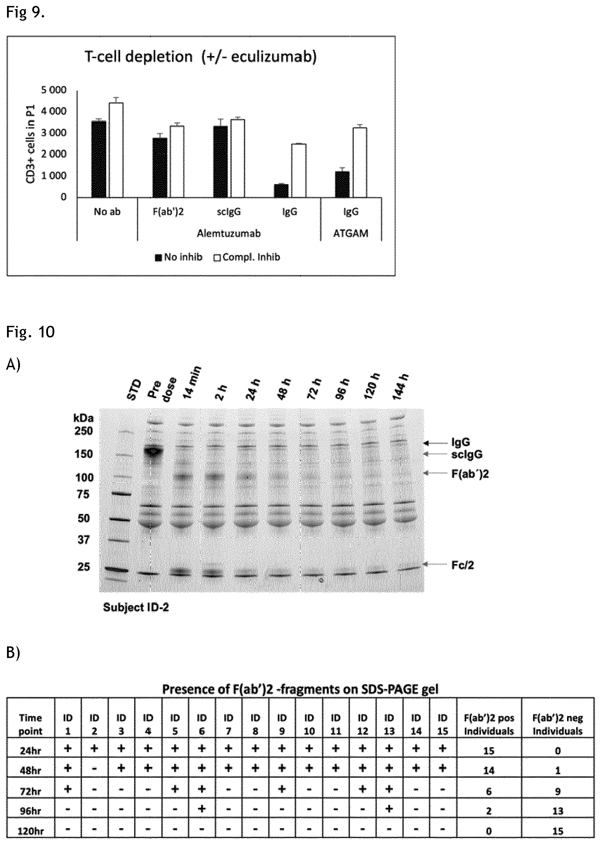

**Fig. 9****. Cytotoxic effect of alemtuzumab can be alleviated by complement inhibition with eculizumab.** Whole blood of a healthy donor was pre-treated with or without the anti-C5 eculizumab (200 µg/mL) and incubated at RT for 30 mins. The samples were treated with either 30 µg/mL intact IgG alemtuzumab, imlifidase-generated F(ab')2, or scIgG alemtuzumab. In addition intact equine IgG ATGAM (200 µg/mL) was also incubated in the presence or absence of the complement inhibitor eculizumab for 4 hours at 37°C, 5% CO₂. The blood samples were RBC-lysed, centrifuged, resuspended, and stained with a PE-conjugated anti-CD3 before analysis by flow cytometer using an Accuri C6.
**Fig. 10****. Clearance of in vivo generated F(ab')2 fragments.**
   Fifteen healthy individuals from a Phase I Study (study number 18-HMedIdeS-15) received one i.v. dose of imlifidase (0,25 mg/kg body weight). Predose samples and post-dose up to 144 hours were collected and analyzed using a 10 well 4-20% Mini-PROTEAN^{®}TGX^{™} Stain Free gel, exemplified for ID-2 (A). Five µL molecular size standard (PPP) were used. Samples were separated at 200 V for 40 min. The gels were activated and scanned using ChemiDoc Imaging system. The gel images from 15 individuals were evaluated using a visual scoring the presence (+) or absence (-) of the F(ab')2 fragments over time (B).

### Brief Description of the Sequences

SEQ ID NO: 1 is the full sequence of IdeS including N terminal methionine and signal sequence. It is also available as NCBI Reference sequence no. WP_010922160.1
SEQ ID NO: 2 is the mature sequence of IdeS, lacking the N terminal methionine and signal sequence. It is also available as Genbank accession no. ADF13949.1
SEQ ID NO: 3 is the full sequence of IdeZ including N terminal methionine and signal sequence. It is also available as NCBI Reference sequence no. WP_014622780.1.
SEQ ID NO: 4 is the mature sequence of IdeZ, lacking the N terminal methionine and signal sequence.
SEQ ID NO: 5 is the sequence of a hybrid IdeS/Z. The N terminus is based on IdeZ lacking the N terminal methionine and signal sequence.
SEQ ID NOs: 6 to 25 are the sequences of exemplary proteases for use in the methods of the invention.
SEQ ID NO: 26 is the sequence of an IdeS polypeptide. Comprises the sequence of SEQ ID NO: 2 with an additional N terminal methionine and a histidine tag (internal reference pCART124).
SEQ ID NO: 27 is the sequence of an IdeZ polypeptide. Comprises the sequence of SEQ ID NO: 4 with an additional N terminal methionine and a histidine tag (internal reference pCART144).
SEQ ID NO: 28 is the sequence of an IdeS/Z polypeptide. Comprises the sequence of SEQ ID NO: 5 with an additional N terminal methionine and a histidine tag (internal reference pCART145).
SEQ ID NO: 29 is the contiguous sequence PLTPEQFRYNN, which corresponds to positions 63-73 of SEQ ID NO: 3.
SEQ ID NO: 30 is the contiguous sequence PPANFTQG, which corresponds to positions 58-65 of SEQ ID NO: 1.
SEQ ID NO: 31 is the contiguous sequence DDYQRNATEAYAKEVPHQIT, which corresponds to positions 35-54 of SEQ ID NO: 3.
SEQ ID NO: 32 is the contiguous sequence DSFSANQEIRYSEVTPYHVT, which corresponds to positions 30-49 of SEQ ID NO: 1.
SEQ ID NOs: 33 to 55 are nucleotide sequences encoding proteases set out above.
SEQ ID NOs: 56 to 69 are the sequences of exemplary proteases for use in the methods of the invention.
SEQ ID NO: 70 is the contiguous sequence NQTN, which corresponds to positions 336-339 of SEQ ID NO: 1.
SEQ ID NO: 71 is the contiguous sequence DSFSANQEIR YSEVTPYHVT, which corresponds to positions 30-49 of SEQ ID NO: 1.
SEQ ID NOs: 72 to 86 are nucleotide sequences encoding polypeptides disclosed herein.
SEQ ID NO: 87 is the sequence SFSANQEIRY SEVTPYHVT, which corresponds to positions 31-49 of SEQ ID NO: 1.
SEQ ID NO: 88 is the sequence DYQRNATEAY AKEVPHQIT, which corresponds to positions 36-54 of the IdeZ polypeptide NCBI Reference Sequence no WP_014622780.1.
SEQ ID NO: 89 is the sequence DDYQRNATEA YAKEVPHQIT, which may be present at the N terminus of a polypeptide of the invention.
SEQ ID NO: 90 is the mature sequence of EndoS (Endoglycosidase of S. pyogenes).
SEQ ID NO: 91 and SEQ ID NO: 92 are further exemplary proteases for use in the methods of the invention. SEQ ID NO: 92 is the same as SEQ ID NO: 91, except it lacks the first twenty residues at the N terminus of SEQ ID NO: 1 consisting of the contiguous sequence DDYQRNATEAY AKEVPHQIT.

### Detailed Description of the Invention

### General

It is to be understood that different applications of the disclosed products and methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "a polypeptide" includes "polypeptides", and the like.

A "polypeptide" is used herein in its broadest sense to refer to a compound of two or more subunit amino acids, amino acid analogs, or other peptidomimetics. The term "polypeptide" thus includes short peptide sequences and also longer polypeptides and proteins. As used herein, the term "amino acid" refers to either natural and/or unnatural or synthetic amino acids, including both D or L optical isomers, and amino acid analogs and peptidomimetics.

The terms "patient" and "subject" are used interchangeably and typically refer to a human. References to IgG typically refer to human IgG unless otherwise stated.

Amino acid identity as discussed above may be calculated using any suitable algorithm. For example the PILEUP and BLAST algorithms can be used to calculate identity or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10. Software for performing BLAST analyses is publicly available through the National Center for Biotechnology Information (This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two polynucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001. Alternatively, the UWGCG Package provides the BESTFIT program which can be used to calculate identity (for example used on its default settings) (Devereux et al (1984) Nucleic Acids Research 12, 387-395).

All publications, patents and patent applications cited herein, whether supra or infra, are hereby incorporated by reference in their entirety.

### Conditioning regimen - lymphocyte depletion or down-modulation

The present invention provides a conditioning regimen for the transplant of a cell to a subject, comprising:
a. administering to the subject an agent comprising IgG molecules, which agent reduces the numbers and/or down-modulates the activity of lymphocytes in the subject;
b. subsequently administering to the subject an enzyme which inactivates serum IgG molecules in the subject; and
c. subsequently administering the transplant to the subject.

Step (a) may be conducted by any suitable method and using any suitable agent. The same agent or combination of agents may be effective to reduce the numbers and/or down-modulate the activity of more than one type of lymphocyte. This may be referred to as lymphocyte depletion or lymphodepletion. The agents may be referred to as lymphodepleting agents. The agents are preferably administered at a dose sufficient to substantially reduce the numbers and/or substantially down-modulate the activity of lymphocytes in the subject. Preferred agents target T lymphocytes and/or B lymphocytes, with T lymphocytes most preferred. The agent may also deplete NK cells and/or non-malignant hematopoietic disease precursors.

Exemplary agents suitable for the depletion of lymphocytes (which may be referred to as lymphodepleting agents) are known in the art and include:
- An anti-CD52 antibody, such as alemtuzumab (also known as Campath-H1). Alemtuzumab is a humanized monoclonal antibody against CD52 and causes depletion of T and B lymphocytes, NK cells, monocytes, granulocytes, and dendritic cells, which all express CD52. Three mechanisms contribute to the depletion of these cells by alemtuzumab: CDC, ADCC and apoptosis. The half-life of alemtuzumab is generally reported at 12 days (288h), but in practice it depends on the concentration of its target. The plasma half-life of alemtuzumab is shorter (around 6 days) in patients with CLL with a large tumor burden due to cytotoxicity of malignant cells and clearance from the blood. In contrast, after successful treatment of a CLL patients, when CD52 levels decrease, the half-life of alemtuzumab increases to 8-21 days. Maintenance of a therapeutic concentration of 100 ng/ml for 56 days after HPSC transplant has been reported. No antidote to accidental overdose of alemtuzumab is known and it is unlikely that alemtuzumab would be removed with hemodialysis due to its size (150 kDa). As such there is currently no effective way to reduce the concentration of alemtuzumab in a patient (such as HPSC transplant recipient) to reduce the risk of relapse and infection. An enzyme as disclosed herein which inactivates serum IgG molecules can be used to achieve this goal, in particular an IgG cysteine protease enzyme as discussed below, preferably imlifidase. Thus, also disclosed herein is the treatment of alemtuzumab overdose by administration of such an enzyme, preferably imlifidase.
- A polyclonal mixture of non-human antibodies directed against human lymphocyte antigens, typically human T and/or B lymphocyte antigens. Various mixtures are well known in the art, and may commonly be referred to by the general term ATG, and specifically rATG (for rabbit originating mixtures) or hATG (for horse originating mixtures). The term "ATG" is taken from the International Non-Proprietary Names for such mixtures, which include (rabbit) anti-human T-lymphocyte immunoglobulin, or (rabbit) anti-human thymocyte globulin. Different "ATG" mixtures will have different antibody profiles and so may have differences in their precise immunosuppressive activities. However, all are a mixture of antibodies intended to bind to various T and B cell antigens, leading to T- and B lymphocyte depletion via (i) apoptosis via activation-induced cell death, (ii) antibody-dependent cell-mediated cytotoxicity, and (iii) complement-dependent cytotoxicity. Currently there are two ATG preparations available for use as lymphodepleting agents in human patients. Both are prepared in rabbits and so may be interchangeably referred to herein by the general term rATG, unless a specific mixture is intended, in which case this will be specified. The two mixtures differ in terms of the immunogen used to raise them and as a consequence each has different antigen specificities. The first mixture may be referred to as ATG-F (for Fresenius) or ATG-G (for Grafalon) and is produced by immunizing rabbits with the T-cell leukemia line Jurkat. The antibody profile in this mixture is rarely against CD3, CD4, CD44, and HLA-DR but targeting CD28, CD29, CD45, CD49, CD98, CD147, and more antibodies directed against CD107 (an antigen expressed on T cells during degranulation following antigenic stimulation). It has a reported half-life of 2-3 days but can be measured in the recipients' blood for up to 5 weeks after infusion. The second mixture may be referred to as Thymoglobulin and is produced by immunizing rabbits with fresh human thymocytes. The antibody profile in this mixture is typically against CD2, CD3, CD4, CD8, CD11a, CD18, CD25, CD44, CD45, HLA.DR, HLA Class I heavy chains and b2-microglobulin. It has a reported half-life of 30 days, with a total dose of 10 mg/kg for 4 days (at Days -4 to - 1) detectable for 8 weeks after infusion and active agent still detectable at Day +28.
- Other agents may include any one or more of:

### Other agents primarily depleting T cells

- a panel of antibodies including anti-CD4, anti-CD8, and anti-CD90; or
- an anti-CD117 antibody; or
- an anti-CD47 antibody; or
- an anti-CD45 antibody; or
- an anti-CD38 antibody (for example daratumumab); or
- abatacept; or
- an immunotoxin targeting T cells; or

### Other agents primarily depleting B cells (optionally including plasma cells)

- an anti-CD20 antibody (such as rituximab);
- an anti-CD 19 antibody;
- an immunotoxin targeting B cells, such as an anti-CD20 immunotoxin (for example MT-3724).

The conditioning regimen may additionally include administration also of a non-IgG based agent that is suitable to reduce the numbers and/or down-modulates the activity of lymphocytes in the subject. For example, any of busulfan, cyclophosphamide, fludarabine, treosulfan, cyclosporine, and tacrolimus may be used to deplete or inactive T cells; and any of bortezomib, fludarabine, and cyclophosphamide may be used to deplete or inactive B cells.

### Conditioning regimen - enzyme

The present invention provides a conditioning regimen for the transplant of a cell to a subject, comprising:
a. administering to the subject an agent comprising IgG molecules, which agent reduces the numbers and/or down-modulates the activity of lymphocytes in the subject;
b. subsequently administering to the subject an enzyme which inactivates serum IgG molecules in the subject; and
c. subsequently administering the transplant to the subject.

The amount of said enzyme administered in step (b) is preferably sufficient to inactivate all or substantially all IgG molecules present in the serum of the subject. If necessary, more than one IgG-inactivating enzyme can be administered in combination, including simultaneously or sequentially, in any order.

The term "serum IgG molecule(s)" or "IgG molecule(s) present in the serum" refers to any gamma immunoglobulin (IgG1, IgG2, IgG3 and IgG4) molecule which is present in human tissue or in circulation prior to a method of the invention being carried out. Such IgG molecules may have been produced endogenously from an individual's B-cells or may be exogenous gamma immunoglobulins which have been administered to a subject prior to the method of the invention being carried out - including any therapeutic IgG molecule of any origin. Inactivation of serum IgG typically means a reduction in the Fc receptor interaction of IgG molecules. The term "Fc receptor" refers to Fc gamma immunoglobulin receptors (FcyRs) which are present on cells. In humans, FcyR refers to one, some, or all of the family of receptors comprising FcyRI (CD64), FcyRIIA (CD32A), FcγRIIB (CD32B), FcyRIIC (CD32C), FcyRIIIA (CD16a) and FcγRIIIB (CD16b). As used herein, the term FcyR includes naturally occurring polymorphisms of FcyRI (CD64), FcyRIIA (CD32A), FcyRIIB (CD32B), FcyRIIC (CD32C), FcyRIIIA (CD16a) and FcγRIIIB (CD16b).

The enzyme used in the method of the invention may be any enzyme which inactivates serum IgG, but is typically an IgG cysteine protease which cleaves IgG such that the antigen binding domains and Fc interacting domains are separated from each other. In such cases, Fc receptor interaction of serum IgG molecules is reduced because the quantity of intact IgG molecules in the serum is reduced. As another example, the enzyme may be an IgG endoglycosidase which cleaves a glycan structure on the Fc interacting domain of IgG, particularly the N-linked bi-antennary glycan at position Asn-297 (Kabat numbering). This glycan structure has a critical role in Fc receptor binding and complement activation. Thus, when it is wholly or partially removed by a protein, this will lead to reduced Fc receptor binding or complement activation by an otherwise intact IgG molecule, as well as also reduced recycling/half-life due to reduced binding to the FcRn. Enzymes suitable for use in the conditioning regimen are discussed in more detail in subsequent sections below.

The enzyme is preferably administered by intravenous infusion, but may be administered by any suitable route including, for example, intradermal, subcutaneous, percutaneous, intramuscular, intra-arterial, intraperitoneal, intraarticular, intraosseous or other appropriate administration routes. The amount of the enzyme that is administered may be between 0.01 mg/kg BW and 2 mg/kg BW, between 0.01 mg/kg BW and 1 mg/kg BW, between 0.01 mg/kg BW and 0.5 mg/kg BW, between 0.01 and 0.3 mg/kg BW, preferably between 0.1 and 0.3 mg/kg BW, most preferably between 0.2 and 0.3 mg/kg BW. Doses of approximately 0.12 mg/kg BW and approximately 0.24 mg/kg BW have been used in clinical settings. A particularly preferred dose is approximately 0.25 mg/kg BW.

The enzyme may be administered on multiple occasions to the same subject, provided that the quantity of anti-drug antibody (ADA) in the serum of the subject which is capable of binding to the enzyme does not exceed a threshold determined by the clinician. The amount of enzyme administered may be increased should a clinician consider this to be appropriate. The quantity of ADA in the serum of the subject which is capable of binding to the protease may be determined by any suitable method, such as an agent specific CAP FEIA (ImmunoCAP) test or a titre assay. If ADA in the subject exceed said threshold, the condition regimen may include administration of an alternative enzyme.

The enzyme may be any of the following:

### IgG cysteine proteases

The IgG cysteine protease for use with the invention is specific for IgG. In preferred embodiments, the protease for use in the methods of the invention is IdeS (Immunoglobulin G-degrading enzyme of *S. pyogenes),* otherwise known as imlifidase. IdeS is an extracellular cysteine protease produced by the human pathogen *S. pyogenes.* IdeS was originally isolated from a group A *Streptococcus* strain of serotype M1, but the *ides* gene has now been identified in all tested group A *Streptococcus* strains. IdeS has an extraordinarily high degree of substrate specificity, with its only identified substrate being IgG. IdeS catalyses a single proteolytic cleavage in the lower hinge region of the heavy chains of all subclasses of human IgG. IdeS also catalyses an equivalent cleavage of the heavy chains of some subclasses of IgG in various animals. IdeS efficiently cleaves IgG to Fc and F(ab')₂ fragments via a two-stage mechanism. In the first stage, one (first) heavy chain of IgG is cleaved to generate a single cleaved IgG (scIgG) molecule with a non-covalently bound Fc/2 molecule. The scIgG molecule is effectively an intermediate product which retains the remaining (second) heavy chain of the original IgG molecule. In the second stage of the mechanism this second heavy chain is cleaved by IdeS to release a F(ab')₂ fragment and a homodimeric Fc fragment. These are the products generally observed under physiological conditions. Under reducing conditions the F(ab')₂ fragment may dissociate to two Fab' fragments and the homodimeric Fc may dissociate into its component monomers. IdeS has been shown to be particularly effective at cleaving IgG in humans. The entire plasma IgG-pool is cleaved within minutes of dosing with IdeS, and IgG levels in blood remain low for more than a week until newly synthesized IgG appeared in plasma. This demonstrates that the entire extracellular IgG pool and not only the plasma pool (i.e. serum IgG molecules) is cleaved by IdeS (Winstedt et al; PloS One 2015; 10(7): e0132011).

SEQ ID NO: 1 is the full sequence of IdeS including the N terminal methionine and signal sequence. It is also available as NCBI Reference sequence no. WP_010922160.1. SEQ ID NO: 2 is the mature sequence of IdeS, lacking the N terminal methionine and signal sequence. It is also available as Genbank accession no. ADF13949.1.

In alternative embodiments, the protease for use in the methods of the invention is IdeZ, which is a IgG cysteine protease produced by *Streptococcus equi ssp. Zooepidemicus,* a bacterium predominantly found in horses. SEQ ID NO: 3 is the full sequence of IdeZ including N terminal methionine and signal sequence. It is also available as NCBI Reference sequence no. WP_014622780.1. SEQ ID NO: 4 is the mature sequence of IdeZ, lacking the N terminal methionine and signal sequence.

In alternative embodiments, the protease for use in the methods of the invention is a hybrid IdeS/Z, such as that of SEQ ID NO: 5. The N terminus is based on IdeZ lacking the N terminal methionine and signal sequence.

In preferred embodiments, the protease for use in the invention may comprise or consist of SEQ ID NO: 2, 4 or 5. Proteases for use in the invention may comprise an additional methionine (M) residue at the N terminus and/or a tag at the C terminus to assist with expression in and isolation from standard bacterial expression systems. Suitable tags include a histidine tag which may be joined directly to the C terminus of a polypeptide or joined indirectly by any suitable linker sequence, such as 3, 4 or 5 glycine residues. The histidine tag typically consists of six histidine residues, although it can be longer than this, typically up to 7, 8, 9, 10 or 20 amino acids or shorter, for example 5, 4, 3, 2 or 1 amino acids.

In further preferred embodiments, the protease for use in the invention may comprise, consist essentially, or consist of the sequence of any one of SEQ ID NOs: 6 to 25. These sequences represent IdeS and IdeZ polypeptides with increased protease activity and/or reduced immunogenicity. Each of SEQ ID NOs: 6 to 25 may optionally include an additional methionine at the N terminus and/or a histidine tag at the C terminus. The histidine tag preferably consists of six histidine residues. The histidine tag is preferably linked to the C terminus by a linker of 3x glycine or 5x glycine residues.

In further preferred embodiments, the protease for use in the invention may comprise, consist essentially, or consist of the sequence of any one of SEQ ID NOs: 56 to 69. These sequences represent IdeS polypeptides with increased protease activity and/or reduced immunogenicity. Each of SEQ ID NOs: 56 to 69 may optionally include an additional methionine at the N terminus and/or a histidine tag at the C terminus. The histidine tag preferably consists of six histidine residues. The histidine tag is preferably linked to the C terminus by a linker of 3x glycine or 5x glycine residues.

In further preferred embodiments, the protease for use in the invention may comprise, consist essentially, or consist of the sequence of any one of SEQ ID NOs: 6 to 25, optionally with up to 3 (such as 1, 2 or 3) amino acid substitutions. Each of SEQ ID NOs: 6 to 25 and variants thereof may optionally include an additional methionine at the N terminus and/or a histidine tag at the C terminus.

In further preferred embodiments, the protease for use in the invention may comprise, consist essentially, or consist of the sequence of any one of SEQ ID NOs: 56 to 69, optionally with up to 3 (such as 1, 2 or 3) amino acid substitutions. Each of SEQ ID NOs: 56 to 69 and variants thereof may optionally include an additional methionine at the N terminus and/or a histidine tag at the C terminus.

The polypeptide of the invention is typically at least 100, 150, 200, 250, 260, 270, 280, 290, 300 or 310 amino acids in length. The polypeptide of the invention is typically no larger than 400, 350, 340, 330, 320 or 315 amino acids in length. It will be appreciated that any of the above listed lower limits may be combined with any of the above listed upper limits to provide a range for the length the polypeptide of the invention. For example, the polypeptide may be 100 to 400 amino acids in length, or 250 to 350 amino acids in length. The polypeptide is preferably 290 to 320 amino acids in length, most preferably 300 to 315 amino acids in length.

The primary structure (amino acid sequence) of a protease of the invention is based on the primary structure of IdeS, IdeZ or IdeS/Z, specifically the amino acid sequence of SEQ ID NO: 2, 4 or 5, respectively. The sequence of a protease of the invention may comprise a variant of the amino acid sequence of SEQ ID NO: 2, 4 or 5, which is at least 80% identical to the amino acid sequence of SEQ ID NO: 2, 4 or 5. The variant sequence may be at least 80%, at least, 85%, preferably at least 90%, at least 95%, at least 98% or at least 99% identical to the sequence of SEQ ID NO: 2, 4 or 5. The variant may be identical to the sequence of SEQ ID NO: 2, 4 or 5 apart from the inclusion of one or more of the specific modifications identified in WO2016/128558 or WO2016/128559. Identity relative to the sequence of SEQ ID NO: 2, 4 or 5 can be measured over a region of at least 50, at least 100, at least 200, at least 300 or more contiguous amino acids of the sequence shown in SEQ ID NO: 2, 4 or 5, or more preferably over the full length of SEQ ID NO: 4 or 5.

The protease for use in the invention may be an IdeS, IdeZ or IdeS/Z polypeptide that comprises a variant of the amino acid sequence of SEQ ID NO:, 2, 4 or 5 in which modifications, such as amino acid additions, deletions or substitutions are made relative to the sequence of SEQ ID NO: 2, 4 or 5. Such modifications are preferably conservative amino acid substitutions. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art.

IgG cysteine protease activity may be assessed by any suitable method, for example by incubating a polypeptide with a sample containing IgG and determining the presence of IgG cleavage products. Suitable methods are described in the WO2016/128559. Suitable assays include an ELISA-based assay, such as that which is described in WO2016/128559. In such an assay, the wells of an assay plate will typically be coated with an antibody target, such as bovine serum albumin (BSA). Samples of the polypeptide to be tested are then added to the wells, followed by samples of target-specific antibody that is antibody specific for BSA in this example. The polypeptide and antibody are allowed to interact under conditions suitable for IgG cysteine protease activity. After a suitable interval, the assay plate will be washed and a detector antibody which specifically binds to the target-specific antibody will be added under conditions suitable for binding to the target-specific antibody. The detector antibody will bind to any intact target-specific antibody that has bound to the target in each well. After washing, the amount of detector antibody present in a well will be proportional to the amount of target-specific antibody bound to that well. The detector antibody may be conjugated directly or indirectly to a label or another reporter system (such as an enzyme), such that the amount of detector antibody remaining in each well can be determined. The higher the potency of the tested polypeptide that was in a well, the less intact target-specific antibody will remain and thus there will be less detector antibody. Typically, at least one well on a given assay plate will include IdeS instead of a polypeptide to be tested, so that the potency of the tested polypeptides may be directly compared to the potency of IdeS. IdeZ and IdeS/Z may also be included for comparison.

Other assays may determine the potency of a tested polypeptide by directly visualizing and/or quantifying the fragments of IgG which result from cleavage of IgG by a tested polypeptide. An assay of this type is also described in WO2016/128559. Such an assay will typically incubate a sample of IgG with a test polypeptide (or with one or more of IdeS, IdeZ and IdeS/Z as a control) at differing concentrations in a titration series. The products which result from incubation at each concentration are then separated using gel electrophoresis, for example by SDS-PAGE. Whole IgG and the fragments which result from cleavage of IgG can then be identified by size and quantified by the intensity of staining with a suitable dye. The greater the quantity of cleavage fragments, the greater the potency of a tested polypeptide at a given concentration. A polypeptide of the invention will typically produce detectable quantities of cleavage fragments at a lower concentration (a lower point in the titration series) than IdeZ and/or IdeS. This type of assay may also enable the identification of test polypeptides that are more effective at cleaving the first or the second heavy chain of an IgG molecule, as the quantities of the different fragments resulting from each cleavage event may also be determined. A polypeptide of the invention may be more effective at cleaving the first chain of an IgG molecule than the second, particularly when the IgG is an IgG2 isotype. A polypeptide of the invention may be more effective at cleaving IgG1 than IgG2.

### IgG endoglycosidases

The enzyme may have IgG endoglycosidase activity, preferably cleaving the glycan moiety at Asn-297 (Kabat numbering) in the Fc region of IgG. An example of such a protein is EndoS (Endo^{g}l^{y}cosidase of *S. pyogenes).* EndoS hydrolyzes the β-1,4-di-*N-*acetylchitobiose core of the asparagine-linked glycan of normally-glycosylated IgG. The mature sequence of EndoS is provided as SEQ ID NO: 90. The agent may be a protein comprising or consisting of the amino acid sequence of SEQ ID NO: 90, or may be a homologue thereof from an alternative bacterium, such as Streptococcus equi or Streptococcus zooepidemicus, or Corynebacterium pseudotuberculosis, Enterococcus faecalis, or Elizabethkingia meningoseptica. The agent may be CP40, EndoE, or EndoF₂.

Alternatively the agent may be a variant of the EndoS protein which comprises or consists of any amino acid sequence which has at least 80%, 85%, 90% or 95% identity with SEQ ID NO: 90 and has IgG endoglycosidase activity. A variant of the EndoS protein may comprise or consist of an amino acid sequence in which up to 1, 2, 3, 4, 5, 10, 20, 30, 40, 50, 60, 70, 80, 90, or more, amino acid substitutions, insertions or deletions have been made relative to the amino acid sequence of SEQ ID NO: 90, provided the variant has IgG endoglycosidase activity. Said amino acid substitutions are preferably conservative. Conservative substitutions are as defined in the preceding section.

Alternatively the agent may be a protein which comprises or consists of a fragment of SEQ ID NO: 90 and has IgG endoglycosidase activity, preferably wherein said fragment is 400 to 950, 500 to 950, 600 to 950, 700 to 950 or 800 to 950 amino acids in length. A preferred fragment consists of amino acids 1 to 409 of SEQ ID NO: 90, which corresponds to the enzymatically active α-domain of EndoS generated by cleavage by the streptococcal cysteine proteinase SpeB. The fragment may be created by the deletion of one or more amino acid residues of the amino acid sequence of SEQ ID NO: 90. Up to 1, 2, 3, 4, 5, 10, 20, 30, 40, 50, 100, 200, 300, 400, 500 or 550 residues may be deleted, or more. The deleted residues may be contiguous with other.

Any fragment or variant of SEQ ID NO: 90 preferably includes residues 191 to 199 of SEQ ID NO: 90, i.e. Leu-191, Asp-192, Gly-193, Leu-194, Asp-195, Val-196, Asp-197, Val-198 and Glu-199 of SEQ ID NO: 90. These amino acids constitute a perfect chitinase family 18 active site, ending with glutamic acid. The glutamic acid in the active site of chitinases is essential for enzymatic activity. Most preferably, therefore, a variant of SEQ ID NO: 90 contains Glu-199 of SEQ ID NO: 90. The variant of SEQ ID NO: 90 may contain residues 191 to 199 of SEQ ID NO: 90 having one or more conservative substitutions, provided that the variant contains Glu-199 of SEQ ID NO: 90.

### Conditioning regime - dose and timing for lymphodepletion and enzymatic inactivation

The dose and timing of administration of a lymphodepleting agent comprising IgG molecules in accordance with step (a) of the method will typically be such that lymphocyte number and/or activity is substantially down-modulated in the individual subject. Suitable dose schedules for known lymphodepleting agents are well known in the art. The agent may be administered as one or more doses, administered spaced out over one or more days. The first dose may be administered at any time, provided the final dose is administered at least one day prior to step (c). The day prior to step (c) may be referred to as D -1, with the day of transplant referred to as D 0. A typical schedule may have a first dose of agent taking place around 14, 13, 12, 11, 10, 8, 7, 6, 5, 4 or 3 days prior to step (c) (may be referred to as D -14, D -13, D -12 etc) followed by, for example, 2 to 7 (for a total of 3 to 8) or 2 to 5 (for a total of 3 to 6) separate, additional doses, typically one dose per day, provided that the final dose is administered at least one day prior to step (c), that is no later than D -1.

The IgG inactivating enzyme is administered in step (b) after the lymphodepleting agent in step (a), typically at a time point sufficiently after step (a) that the lymphodepleting agent has had the desired effects on lymphocyte numbers and/or activity, but sufficiently in advance of the subsequent cell transplant that inactivation by the enzyme will prevent negative effects that may be caused by the agent. The timing of step (b) is typically at least 1 day after the conclusion of step (a), that is 1 day after the final dose of agent is administered in step (a). Step (b) may take place on the same day as step (c), that is on D 0, but may preferably be at least 2, 3, 4, 5 or 6 hours prior to step (c). The dose and timing of administration of the IgG inactivating enzyme will typically be sufficient to inactivate substantially all serum IgG molecules in the individual subject (i.e. including all of the lymphodepleting agent comprising IgG molecules), prior to administration of the transplant in step (c). Suitable dose schedules for known IgG inactivating enzymes are well known in the art.

By way of illustration, it is preferred that: where the lymphodepleting agent is alemtuzumab, the inactivating enzyme should be administered at a time and dose such that the level of intact agent present in the subject immediately prior to transplant is reduced to less than 0. 1µg/ml; where the lymphodepleting agent is ATG-G, the inactivating enzyme should preferably be administered at a time and dose such that the level of intact agent present in the subject immediately prior to transplant is reduced to less than 1µg/ml; and where the lymphodepleting agent is Thymoglobulin, the inactivating enzyme should preferably be administered at a time and dose such that the level of intact agent present in the subject immediately prior to transplant is reduced to less than 0.3µg/ml,

Dose and timing schedules for each of various exemplary lymphodepleting agents comprising IgG molecules, and an exemplary IgG inactivating enzyme, are shown below to illustrate the invention. Timing is shown relative to the day of transplant in step (c).

| Step (a) | | | | |
|---|---|---|---|---|
| Lymphodepleting agent | | **Alemtuzumab** | **ATG-G** | **Thymoglobulin** |
| Total dose between | | 0.5 - 250 mg/kg BW | 5-80 mg/kg BW | 0.5-15 mg/kg BW |
| | - preferably | 0.7 - 200 mg/kg BW | 5-60 mg/kg BW | 0.8 - 10 mg/kg BW |
| | - more preferably | 0.96 - 144 mg/kg BW | 20 - 45 mg/kg BW | 5 -10 mg/kg BW |
| | - preferred | Approx 1 mg/kg BW | Approx 30 mg/kg BW | Approx 10 mg/kg BW |
| Preferred administration protocol | | 3 - 8 equal doses (preferably 5) typically evenly spaced over around 4-5 days | 3 - 6 doses (preferably 4) typically evenly spaced over around 3-4 days, first dose may be a lower loading dose | 3 - 6 doses (preferably 4) typically evenly spaced over around 3-4 days, first dose may be a lower loading dose |
| Timing of last dose | | Any of D -9 to D -1 | Any of D -9 to D -1 | Any of D -9 to D -1 |
| Step (b) | | | | |
| Inactivating enzyme | | imlifidase | imlifidase | imlifidase |
| Dose between | | 0.01 - 2 mg/kg BW | 0.01 - 2 mg/kg BW | 0.01 - 2 mg/kg BW |
| | - preferably | 0.01 - 1 mg/kg BW | 0.01 - 1 mg/kg BW | 0.01 - 1 mg/kg BW |
| | - preferred | Approx 0.25 mg/kg BW | Approx 0.25 mg/kg BW | Approx 0.25 mg/kg BW |

| Preferred administration protocol | | Single dose | Single dose | Single dose |
|---|---|---|---|---|
| Timing | | At least 1 day after final dose of step (a), at any of: D -6 to D 0 - typically D -2 | At least 1 day after final dose of step (a), at any of: D -6 to D 0 typically D -2 | At least 1 day after final dose of step (a), at any of: D -6 to D 0 typically D -2 |

A particular exemplary dose and timing schedule for each of the various exemplary lymphodepleting agents comprising IgG molecules and an exemplary IgG inactivating enzyme is shown below. Timing is shown relative to the day of cell transplant.

| Step (a) | | | |
|---|---|---|---|
| Lymphodepleting agent | **Alemtuzumab** | **ATG-G** | **Thymoglobulin** |
| Total dose | Approx 1 mg/kg BW | Approx 30 mg/kg BW | Approx 10 mg/kg BW |
| Preferred administration protocol | 5 daily doses of 0.2 mg/kg BW (start on D -13) | 4 doses as follows: | 4 doses as follows: |
| | | 1× 1 mg/kg (d-12) | 1× 1 mg/kg (d-12) |
| | | 1× 9 mg/kg (d-11) | 1× 3 mg/kg (d-11) |
| | | 1× 10 mg/kg (d-10) | 1× 3 mg/kg (d-10) |
| | | 1× 10 mg/kg (d-9) | 1× 3 mg/kg (d-9) |
| Timing of last dose | D -9 | D -9 | D -9 |

| Step (b) | | | |
|---|---|---|---|
| Inactivating enzyme | imlifidase | imlifidase | imlifidase |
| Dose | Approx 0.25 mg/kg BW | Approx 0.25 mg/kg BW | Approx 0.25 mg/kg BW |
| Preferred administration protocol | Single dose (infusion) | Single dose (infusion) | Single dose (infusion) |
| Timing | D -2 | D -2 | D -2 |

### Conditioning regimen - additional steps

The conditioning regimen may additionally comprise one or more of:
(i) administration to the subject of a non-lethal dose of irradiation and/or any agent which depletes the subject's HPSC;
(ii) administration any other agent or regimen which modulates (e.g. reduces) the activity of the immune system , e.g., inhibitors of complement, inhibitors of cytokines, inhibitors of innate immune cells, inducers of tolerance.

Step (i) typically involves administering a dose of radiation which is sufficient to partially or totally eradicate (or ablate) the bone marrow of the subject. It is particularly applicable when the donor cells are HPSCs. Partial eradication by irradiation may be preferred since the side effects are typically less severe and also because it is desirable to retain some recipient bone marrow. The ablation of recipient bone marrow creates space in the bone marrow for engraftment of donor cells, but also depletes lymphocytes in the subject and thus also reduces immune system activity in the same manner as step (a). As such, the conditioning regimen preferably includes at least (a), but most preferably includes at least (a) and (i). Alternatively, it may be preferred in step (a) to use an irradiation free approach to depletion of subject HSPCs, such as administration of anti-CD 117 and/or anti-CD47. This will create space for engraftment of donor HSPCs, but without some of the undesirable side-effects of irradiation. In addition to HSPC depletion, the subject may also optionally receive an infusion of donor CD8-alpha cells, which may increase the frequency of stable chimerism in sensitized recipients. Donor T cell infusion may promote donor HSPC engraftment by reducing survival of host T cells.

### Method for inducing hematopoietic chimerism

The present invention provides a method for the induction of hematopoietic chimerism in a subject, the method comprising conducting the conditioning regimen of the invention and subsequently administering HSPC to the subject in an amount sufficient and under conditions suitable to induce hematopoietic chimerism in the subject. The method may alternatively be described as a method for the stable transplantation of HSPC. The HSPC may be autologous (the patient's own cells are used) or syngeneic (the cells are from a genetically identical twin), or they may allogeneic (the cells come from a separate, non-identical donor).

Immune complications which reduce the likelihood of successful engraftment of HSPC in the recipient are most significant for allogeneic cells and thus the method of the invention is of greatest benefit with such cells. However, immune complications can occur even with autologous cells if there is expression of a product to which the recipient has not previously been exposed. If an autologous cell has been genetically modified to express a gene therapy, the cell may be sufficiently altered to provoke an immune response. For example there may be an immune response to the expressed gene therapy product. Similar would apply if the HSPC has been genetically modified to express a different HLA type which is not matched to the HLA of the recipient. Therefore the HSPC are preferably allogeneic, or are genetically modified autologous or syngeneic cells, for example Chimeric antigen receptor (CAR) T-cells. The HSPC are most preferably allogeneic. In a particularly preferred embodiment, the HSPC are from a donor who is also the donor of another organ or tissue which is to be transplanted into the recipient. That is, the same donor provides both the HSPC and the other cell, organ or tissue.

HSPC are found in the bone marrow of adults, especially in the pelvis, femur, and sternum. They are also found in umbilical cord blood and, in small numbers, in peripheral blood. HSPC may be harvested from these locations using any suitable technique established in the art.

For example, HSPC may be harvested from human bone marrow by aspirating directly from the centre of a bone of the donor with a large needle. The posterior iliac crest is the usual site of harvest. The technique is referred to as a bone marrow harvest and may be performed under local or general anesthesia. When the administered HSPC are derived from the bone marrow of the donor, the administration of HSPC may be described as a bone marrow transplant (BMT).

HSPC may be harvested from umbilical cord blood shortly after the birth of an infant. The umbilical cord is double-clamped from the umbilicus and transacted between clamps. The umbilical cord vein is then punctured under sterile conditions, and the blood flows freely by gravity into an anticoagulated sterile closed harvesting system, form which the HSPC may be isolated.

HSPC may be harvested from peripheral blood, typically by apheresis. However, because numbers of HSPC in peripheral blood are normally low, it is first necessary to mobilize HSPCs from the bone marrow. In a healthy donor, this can be achieved by administration of Granulocyte colony-stimulating factor (G-CSF). Alternative strategies may be required if the donor is not healthy. This may frequently be the case if the intended HSPC transplant is autologous.

HSPC are preferably used as quickly as possible after harvesting (that is fresh), but may be cryopreserved for storage prior to thawing for use in the method of the invention. Cryopreservation typically includes volume depletion by removal of red cells and plasma. The quantity of stem cells in the harvest may be quantified, e.g. by flow cytometric analysis of a sample, to establish the proportion of cells which are positive for CD34 (a marker for stem cells).

The HSPC may be administered to the subject by any suitable method. A preferred method is infusion, typically through a central line. The patient may be kept in highly clean or sterile conditions, such as in a room with high-efficiency particulate air (HEPA) filters under positive pressure, before, during and after the infusion to reduce the risk of infection.

The method may be monitored to determine that the HSPC transplant has successfully resulted in hematopoietic chimerism. This is achieved by determining the proportion of donor-derived hematopoietic cells present in a blood sample taken from the subject after a particular time interval, typically 28 days after administration of the HSPC. For example, hematopoietic chimerism may be defined as achieved if at least 5% of the lymphocytes and/or myeloid cells in the sample are found to be donor-derived, preferably if at least 5% of the lymphocytes in the sample are found to be donor-derived. The chimerism is described as mixed if no more than 90% of the lymphocytes and/or myeloid cells in the sample are found to be donor-derived (that is at least 10% are still derived from the recipient), preferably if no more than 90% of the lymphocytes in the sample are found to be donor-derived (that is at least 10% of lymphocytes are still derived from the recipient). The chimerism may be described as total if 98% or more of the lymphocytes and/or myeloid cells in the sample are found to be donor-derived. Mixed chimerism is typically preferred for the methods of the invention, because the recipient will have a greater level of immunocompetence. However, full chimerism may be beneficial in some circumstances, for example in the treatment of cancers such as leukemia where the goal is to eliminate host cells with the potential to cause cancer recurrence, replacing them with the transplanted HSPC.

The proportion of donor and recipient derived cells in a sample may be determined by any suitable method in the art, such as flow cytometric analysis as described in the Examples. Real-time PCR may also be used. Other methods are discussed in Agrawal et al Bone Marrow Transplantation 2004 (34) p-12.

### Methods of treating or preventing a disease or condition

The present invention provides a method for the prevention or treatment of a disease or condition in a subject. The method comprises administering a cell transplant to the subject, wherein said administering comprises the conditioning regimen of the invention. Expressed another way, the invention also provides a method for the prevention or treatment of immune rejection of a cell transplant, the method comprising the conditioning regimen of the invention.

The cell transplanted may be of any type, including HSPC. The HSPC may be a genetically modified HPSC, such that the method effectively comprises administering a gene therapy to the subject in which the HSPC is the vector. The cell to be transplanted may originate from a different species to the recipient, that is it may be a xenotransplant. Suitable species for xenotransplantation into human recipients may include pigs or non-human primates. In such cases the HSPC may be genetically modified to aid with tolerance to the transplant. The cell that is a xenotransplant may also be genetically modified.

The subject to be treated may be sensitized or highly sensitized. By "sensitized" it is meant that the subject has developed antibodies to human major histocompatibility (MHC) antigens (also referred to as human leukocyte antigens (HLA)). The anti-HLA antibodies originate from allogeneically sensitized B-cells and are usually present in patients that have previously been sensitized by blood transfusion, previous transplantation or pregnancy. Achieving hematopoietic chimerism in sensitized patients may reverse allosensitization, through the generation of specific tolerance in T and B cells, resulting in a reduction of donor specific immune responses such as DSA.

Whether or not a potential transplant recipient is sensitized may be determined by any suitable method. For example, a Panel Reactive Antibody (PRA) test may be used to determine if a recipient is sensitized. A PRA score >30% is typically taken to mean that the patient is "high immunologic risk" or "sensitized". Alternatively, a cross match test may be conducted, in which a sample of the potential transplant donor's blood is mixed with that of the intended recipient. A positive cross-match means that the recipient has antibodies which react to the donor sample, indicating that the recipient is sensitized and transplantation should not occur. Cross-match tests are typically conducted as a final check immediately prior to transplantation.

The method may be for the prevention or treatment of any disease or condition that is treated by HSPC transplant. Diseases or conditions typically treated by HSPC transplant may be acquired or congenital. Acquired diseases or conditions that may be treated by HSPC transplant include:
- Hematological malignancies such as leukemias (for example Acute lymphoblastic leukemia (ALL), Acute myeloid leukemia (AML), Chronic lymphocytic leukemia (CLL), Chronic myelogenous leukemia (CML); lymphomas (for example Hodgkin's disease, Non-Hodgkin's lymphoma) and Myelomas (for example, Multiple myeloma (Kahler's disease)).
- Solid tumor cancers, such as Neuroblastoma, Desmoplastic small round cell tumor, Ewing's sarcoma, Choriocarcinoma.
- Hematologic diseases such as phagocyte disorders (for example Myelodysplasia); Anemias (for example Paroxysmal nocturnal hemoglobinuria (PNH; severe aplasia), Aplastic anemia, Acquired pure red cell aplasia); Myeloproliferative disorders (for example Polycythemia vera, Essential thrombocytosis, Myelofibrosis).
- Metabolic disorders such as amyloidosis (for example Amyloid light chain (AL) amyloidosis).
- Environmentally-induced diseases such as radiation poisoning.
- Viral diseases such as Human T-lymphotropic virus (HTLV) or Human Immunodeficiency Viruses (HIV).
- Autoimmune diseases, such as multiple sclerosis.

Congenital diseases or conditions that may be treated HSPC transplant include:
- Lysosomal storage disorders such as Lipidoses - disorders of lipid storage - (for example Neuronal ceroid lipofuscinoses, Infantile neuronal ceroid lipofuscinosis (INCL, Santavuori disease,), Jansky-Bielschowsky disease (late infantile neuronal ceroid lipofuscinosis)); Sphingolipidoses (for example Niemann-Pick disease, Gaucher disease); Leukodystrophies (for example Adrenoleukodystrophy, Metachromatic leukodystrophy, Krabbe disease (globoid cell leukodystrophy)); Mucopolysaccharidoses (for example Hurler syndrome (MPS I H, α-L-iduronidase deficiency), Scheie syndrome (MPS I S), Hurler-Scheie syndrome (MPS I H-S), Hunter syndrome (MPS II, iduronidase sulfate deficiency), Sanfilippo syndrome (MPS III), Morquio syndrome (MPS IV), Maroteaux-Lamy syndrome (MPS VI), Sly syndrome (MPS VII)); Glycoproteinoses (for example Mucolipidosis II (I-cell disease), Fucosidosis, Aspartylglucosaminuria, Alpha-mannosidosis; or Others (for example Wolman disease (acid lipase deficiency)
- Immunodeficiencies, such as T-cell deficiencies (for example Ataxia-telangiectasia, DiGeorge syndrome); Combined T- and B-cell deficiencies (for example Severe combined immunodeficiency (SCID), all types); Wiskott-Aldrich syndrome; Phagocyte disorders (for example Kostmann syndrome, Shwachman-Diamond syndrome); Immune dysregulation diseases (for example Griscelli syndrome, type II); Innate immune deficiencies (for example NF-Kappa-B Essential Modulator (NEMO) deficiency
- Hematologic diseases, such as Hemoglobinopathies (for example Sickle cell disease, β thalassemia major (Cooley's anemia)); Anemias (for example Aplastic anemia, Diamond-Blackfan anemia, Fanconi anemia); Cytopenias (for example Amegakaryocytic thrombocytopenia); and Hemophagocytic syndromes (for example Hemophagocytic lymphohistiocytosis (HLH)).

Where the HSPC are genetically modified to administer a gene therapy, the method of the invention may be for the prevention or treatment of the disease or condition to which said gene therapy is directed.

The invention also provides an enzyme which inactivates serum IgG molecules in a subject for use in a method for the prevention or treatment of a disease or condition, wherein the method is as described above.

The invention also provides the use of an enzyme which inactivates serum IgG molecules in a subject in the manufacture of a medicament, wherein the medicament is for the prevention or treatment of a disease or condition in a method as described above.

### Production of polypeptides

The enzymes used in the methods of the invention are polypeptides and may be produced by any suitable means. For example, a polypeptide may be synthesised directly using standard techniques known in the art, such as Fmoc solid phase chemistry, Boc solid phase chemistry or by solution phase peptide synthesis. Alternatively, a polypeptide may be produced by transforming a cell, typically a bacterial cell, with a nucleic acid molecule or vector which encodes said polypeptide. Production of enzyme polypeptides by expression in bacterial host cells is described and exemplified in WO2016/128558 and WO2016/128559.

### Compositions and formulations comprising polypeptides

The present invention also provides compositions comprising an enzyme for use in the methods of the invention. For example, the invention provides a composition comprising one or more polypeptides, and at least one pharmaceutically acceptable carrier or diluent. The carrier(s) must be 'acceptable' in the sense of being compatible with the other ingredients of the composition and not deleterious to a subject to which the composition is administered. Typically, carriers and the final composition are sterile and pyrogen free.

Formulation of a suitable composition can be carried out using standard pharmaceutical formulation chemistries and methodologies all of which are readily available to the reasonably skilled artisan. For example, the enzyme can be combined with one or more pharmaceutically acceptable excipients or vehicles. Auxiliary substances, such as wetting or emulsifying agents, pH buffering substances, reducing agents and the like, may be present in the excipient or vehicle. Suitable reducing agents include cysteine, thioglycerol, thioredoxin, glutathione and the like. Excipients, vehicles and auxiliary substances are generally pharmaceutical agents that do not induce an immune response in the individual receiving the composition, and which may be administered without undue toxicity. Pharmaceutically acceptable excipients include, but are not limited to, liquids such as water, saline, polyethylene glycol, hyaluronic acid, glycerol, thioglycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients, vehicles and auxiliary substances is available in Remington's Pharmaceutical Sciences (Mack Pub. Co., N.J. 1991).

Such compositions may be prepared, packaged, or sold in a form suitable for bolus administration or for continuous administration. Injectable compositions may be prepared, packaged, or sold in unit dosage form, such as in ampoules or in multi-dose containers containing a preservative. Compositions include, but are not limited to, suspensions, solutions, emulsions in oily or aqueous vehicles, pastes, and implantable sustained-release or biodegradable formulations. Such compositions may further comprise one or more additional ingredients including, but not limited to, suspending, stabilizing, or dispersing agents. In one embodiment of a composition for parenteral administration, the active ingredient is provided in dry (for e.g., a powder or granules) form for reconstitution with a suitable vehicle (e. g., sterile pyrogen-free water) prior to parenteral administration of the reconstituted composition. The compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono-or di-glycerides.

Other parenterally-administrable compositions which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt. The compositions may be suitable for administration by any suitable route including, for example, intradermal, subcutaneous, percutaneous, intramuscular, intra-arterial, intraperitoneal, intraarticular, intraosseous or other appropriate administration routes. Preferred compositions are suitable for administration by intravenous infusion.

### Kits

The invention also provides a kit for carrying out the methods described herein. The kit of the invention may include an enzyme or a composition comprising an enzyme, as described above. The kit may include means for administering the enzyme or composition to a subject. The kit may include instructions for use of the various components in any method as described herein.

### EXAMPLES

Unless indicated otherwise, the methods used are standard biochemistry and molecular biology techniques. Examples of suitable methodology textbooks include Sambrook et al., Molecular Cloning, A Laboratory Manual (1989) and Ausubel et al., Current Protocols in Molecular Biology (1995), John Wiley and Sons, Inc.

### Example 1 - optimal spacing of imlifidase and rATG

### Background

Imlifidase (conditionally authorised in the EU for kidney transplant desensitization) is a cysteine protease which cleaves all subclasses of human and rabbit IgG to a F(ab')₂ fragment and a dimeric Fc fragment. Rabbit anti-thymocyte globulin (rATG) is a depleting antibody therapy approved for induction in kidney transplantation (it effects a large reduction in circulating T-lymphocytes). Antibody-based therapies such as rATG may be inactivated if given with imlifidase. The purpose of this study was to investigate the cleavage of rATG by imlifidase.

### Methods

The cleavage pattern of rATG was investigated with sera from healthy subjects (n=11) treated with 0.25 mg/kg imlifidase (EudraCT number: 2019-002770-31). Serum samples were incubated with a fixed, clinically relevant, concentration of 50 µg/mL rATG (commonly observed after a dose of 1.5 mg/kg), for 2 hours at 37°C. Serum samples were collected pre-imlifidase through 14 days post-imlifidase and were analyzed using SDS-PAGE and Western blot, developed with a goat anti-rabbit IgG, F(ab')₂ specific antibody to evaluate the cleavage of rATG. Imlifidase concentration was analyzed using a validated electroluminescence immunoassay based on MSD technology.

### Results

The imlifidase serum concentration in the subjects declined rapidly and at 96 hours the mean concentration was 0.5 µg/mL, though with a large individual variation, <0.1-1.8 µg/mL (Figure 1). At this timepoint the level of imlifidase activity had decreased sufficiently to avoid complete cleavage of rATG in 8 of 11 subjects (Figure 2).

### Conclusions

rATG is rapidly cleaved and inactivated by imlifidase in human serum.

### Example 2 - use of imlifidase in preconditioning regimens with different antibody-based lymphodepleting agents

Cell therapies such as hematopoietic stem cell transplantation (HSCT) can be curative treatment options for patients with malignant diseases, e.g., acute lymphoblastic leukemia (ALL), acute myeloid leukemia (AML), and for patients with non-malignant diseases, e.g., immune deficiencies, Thalassemia and sickle cell disease. rATG agents (such as ATG-G or Thymoglobulin) and anti-CD52 antibodies such as alemtuzumab are used prior to allogeneic T-cell depleted stem cell transplantation to allow a reduced intensity conditioning, leading to a significant reduction of graft versus host disease (GvHD) and engraftment failure. High doses of these drugs, however, are associated with delayed immune recovery due to their long half-life and will enhance the risk of relapse and infection. Therefore, a means to protect donor lymphocytes, particularly donor NK cells, from any remaining lymphodepleting antibodies may be beneficial.

Imlifidase is a cysteine protease derived from Streptococcus pyogenes which effectively hydrolyzes human and rabbit IgG, in the lower hinge region, into a F(ab')2 fragment and a dimeric Fc/2-fragment (2^{∗}Fc/2). The resulting F(ab')2 fragment retains full binding capacity to its antigens but the IgG-dependent Fc-dependent effector mechanisms, such as cell-mediated toxicity (CDC), antibody-dependent cell-mediated cytotoxicity (ADCC) and antibody-dependent cellular phagocytosis (ADCP) are inactivated.

Since the mechanisms of action of ATG-G, Thymoglobulin and alemtuzumab are, to a large extent, Fc-mediated, imlifidase (or similar enzymes) may be used as a tool to inactivate these drugs and eliminate toxicity, especially towards NK cells, before HSCT. Such a reduced intensity conditioning practice can be particularly useful in more vulnerable patients such as young children and elderly. In this case, imlifidase-inactivated high doses of ATG-G, Thymoglobulin and alemtuzumab, leads to reduction of risk of GvHD, engraftment failure and relapse while boosting immune recovery by eliminating toxicity towards NK cells thus fostering a quicker immune recovery. The suitability of imlifidase (or similar enzymes) in this context is demonstrated by the experiments performed here.

### Materials and Methods

### SDS-PAGE analysis of antibody cleavage by imlifidase

ATG-G, alemtuzumab, ATGAM, and thymoglobulin (1 mg/mL) were diluted in culture medium and incubated with 20 µg/mL imlifidase at 37°C for 2 hours. The samples were diluted 10x in PBS and further diluted in 4X SDS sample buffer and incubated at 92°C for 3 min. The samples were run on 4-20% Mini-PROTEAN^{®} tgx PRECAST SDS-PAGE gels (200V, 40 min), using 1× Tris/Glycine/SDS-buffer. As size standard, 5 µL Precision Plus MW standard (#161-0363 Bio-Rad) was used. The gels were activated for 2.5 min and exposed for 1 s using a Gel Doc EZ Imager system (Bio-Rad) and Image Lab 6.0 software (Bio-Rad).

### Preparation of F(ab')2 and 2*Fc/2-fragments for rATG and alemtuzumab

rATG and alemtuzumab were hydrolyzed, using the FragIT Kit containing immobilized IdeS coupled to agarose beads on spin-columns, for 45 min at 37°C on a shaker. The antibody fragments were collected after centrifugation and transferred to rabbit- or human-specific Fc-binding spin-columns to separate Fc- and F(ab')₂-fragments. The concentrations of the fragment fractions were determined by NanoDrop.

### Cell lines used in the cytotoxicity assays

K562 is a target cell line from a CML patient in blast crisis carrying the BCR-ABL1 e14-a2 (b3-a2) fusion gene; The feeder cell line K562-mbIL15-4-1BBL express membrane-anchored IL15. Both cell lines are grown in RPMI 1640 medium with FCS (10%), L-Glut (2 mM) and Pen/Strep (100 µg/ml).

### Cytotoxicity assay of full length rATG or alemtuzumab and their respective F(ab')₂-2*Fc/2- fragments on NK cells against K562 cells.

The expanded NK cells (eNKs) were generated by incubating PBMCs from healthy donors with IL-2 (50 U/ml) in the presence of feeder cells (K562-mbIL15-4-1BBL) for 14 days. These cells can be frozen in aliquots for posterior use in additional experiments. Thawed eNKs were cultivated for 16h in RPMI-1640 medium supplemented with IL-2 (100 U/ml) and human AB serum (10%), then incubated with or without full length ATG-G or alemtuzumab or their respective F(ab')₂- or 2^{∗}Fc/2- fragments and human AB serum (25%) for 6h. These cells were subsequently used as effector (E) cells against calcein-labeled K562 wild type target cells (T) in calcein release assays for 2 hours on E:T, 10:1 proportion. The amount of released calcein corresponds to the NK cell-mediated-cytotoxicity. Concentrations ofF(ab')₂- and 2^{∗}Fc/2-fragments were chosen according to the percentage of molecular weight of full length alemtuzumab.

Specific lysis (%) was calculated as follows: [(test release minus spontaneous release)/(maximum release minus spontaneous release)] ^{∗} 100. Spontaneous release represents the release of calcein from the target cells in medium alone and maximum release represents the calcein release from target cells lysed in medium plus 1% Triton X-100 (each measured in triplicates). Spontaneous release (%) was calculated as follows: [(spontaneous release minus background)/(maximum release minus background)] ^{∗} 100. Background consists of medium plus the supernatant of calcein-labeled cells.

### Alemtuzumab and Thymoglobulin CD3+ cytotoxicity in whole blood

Peripheral blood was collected from a healthy volunteer in sodium heparin tube (BD-Plymouth). The whole blood was incubated at 37°C, 5% CO₂ for 4 and 16 hours with either alemtuzumab (Campath, anti-CD52, Genzyme) or Thymoglobulin (ATG, Genzyme), at final antibody concentrations of 100, 10, 1, 0.1 and 0 µg/mL. RBC were lysed upon sample incubation with Lysis Buffer (#349202, BD) for 5 minutes. The samples were centrifuged, and the supernatant discarded. The pellets were washed in FACS buffer and centrifuged at 1200 rpm for 3 min. The pellets were resuspended in 50 µL PE-conjugated anti-CD3 antibody (Cat. No. MA1-10179, Thermo Scientific) and incubated at 4°C for 30 mins. FACS-buffer was added and the samples were analyzed on Accuri C6 flow cytometer.

### FACS and SDS-PAGE analysis of alemtuzumab cleavage by imlifidase in whole blood

Whole blood was incubated with 30, 3, 0.3, 0.03 and 0 µg/mL imlifidase for 6 hours at 37°C, 5% CO₂. PBS/BSA or alemtuzumab (Campath, anti-CD52, Genzyme) were added to the samples at a final concentration of 100 µg/mL and incubated overnight at 37°C, 5% CO₂. Part of these samples was directly used for SDS-PAGE where one µL plasma was mixed with 40 µL 2x SDS-loading buffer, heated for 5 min at 92°C, from which 5 µL were separated on a 4-20% SDS-PAGE gel (200 V, 40 min). Precision Plus MW standard, 5 µL, was used as control. The remaining of the samples were lysed and labelled with PE-conjugated anti-CD3 antibody (Cat. No. MA1-10179, Thermo Scientific) at 4°C for 30 mins and analyzed on Accuri C6 flow cytometer.

### FACS and SDS-PAGE analysis of alemtuzumab cleavage by imlifidase in whole blood.

Whole blood was incubated with imlifidase (30, 15, 7.5, 3.8, 1.9, 0.94, 0.47, 0.23, 0.12, 0.056 and 0 µg/mL) for 6 hours at 37°C, 5% CO₂. PBS/BSA or alemtuzumab (Campath, anti-CD52, Genzyme) were added to the samples at a final concentration of 100 µg/mL and incubated overnight at 37°C, 5% CO₂. Plasma aliquots of one µL were mixed with 40 µL 2x SDS-loading buffer, heated 5 min at 92°C, from which 5 µL were separated on a 4-20% SDS-PAGE gel (200V, 40 min). Precision Plus MW standard, 5 µL, was used as control. The remaining of the samples were RBC-lysed and labelled with PE-conjugated anti-CD3 antibody (Cat. No. MA1-10179, Thermo Scientific) at 4°C for 30 mins and analyzed on Accuri C6 flow cytometer.

### Efficacy test of horse ATGAM IgG after imlifidase treatment

Whole blood from a healthy donor was treated in vitro for 6 hours with imlifidase (30, 15,7.5, 3.8, 1.9, 0.94, 0.47, 0.23, 0.12, 0.059 and 0 µg/mL) before further incubation with 1% horse serum in PBS or 500 µg/mL ATGAM (equine Anti-thymocyte Globulin) for 16 hours at 37°C, 5% CO₂. One µL sample aliquots were mixed with 40 µL 2x SDS-loading buffer, heated for 5 min at 92°C. Five µL were separated on a 4-20% SDS-PAGE gel (200V, 40 min). Precision Plus MW standard (5 µL) was used as control. The remaining of the ATGAM and imlifidase-treated samples were further RBC-lysed and labelled with PE-conjugated anti-CD3 antibody (Cat. No. MA1-10179, Thermo Scientific) at 4°C for 30 mins and analyzed on Accuri C6 flow cytometer.

### Evaluating the blocking of CDC by alemtuzumab with eculizumab in a whole blood assay

Whole blood of a healthy donor was pre-treated with or without the anti-C5 eculizumab (Soliris) (200 µg/mL) and incubated at RT for 30 mins. The samples were further treated with either 30 µg/mL alemtuzumab (Campath, anti-CD52) or 200 µg/mL ATGAM for 4 hours at 37°C, 5% CO₂, in the presence and absence of complement inhibitor. The samples were RBC-lysed, centrifuged, resuspended, and stained with a PE-conjugated anti-CD3 antibody (Cat. No. MA1-10179, Thermo Scientific) at 4°C for 30 mins and analyzed on Accuri C6 flow cytometer.

### Phagocytosis (ADCP) Assay

Alemtuzumab (Campath, anti-CD52, Genzyme), was pre-treated with different concentrations of imlifidase to generate F(ab')2, scIgG alemtuzumab. Samples were run on SDS-PAGE to confirm cleavage status.

Target NuDUL-1 cells were washed in PBS and stained with calcein at RT for 10 min in the dark. Cells were washed and resuspendded in R10 medium at a concentration of 1 × 10⁶ cells/mL. THP-1 effector (E) cells, cultured in R10 medium, were washed and resuspended in PBS before staining with FarRed. After two washes, the cells were resuspended in R10 medium (1 × 10⁶ cells/mL).

The NuDUL1 target cells were seeded in V-shaped wells (96-well plate), together with imlifidase generated F(ab')2, scIgG, or intact alemtuzumab at a final concentration of 10 µg/mL and incubated for 20 min prior to incubation with THP-1 effector cells. A sample without alemtuzumab was also prepared as negative control.

The THP-1 effector cells (200 000/well) and target cells (10 000/well) were incubated at 37°C, 5% CO₂ for 90 min. The plate was centrifuged and the supernatant discarded. Resuspended cells were fixed with 4% PFA for 3 min before being resuspended in PBS (+0.5% BSA) after removal of the supernatant, and transfer to FACS tubes for analysis using the Accuri C6 flow cytometer.

### In Vivo F(ab')2 elimination after imlifidase treatment

Fifteen healthy individuals from a Phase I Study (study number 18-HMedIdeS-15) receiving one i.v. dose of 0,25 mg/kg body weight imlifidase. Pharmacodynamic samples were collected predose and up to 144 hours post dose and analyzed using a 10 well 4-20% Mini-PROTEAN^{®}TGX^{™} Stain Free gel. The serum samples were diluted 1:10 in PBS and 10 µL diluted serum samples mixed with 30 µL 2× SDS-PAGE loading buffer. Samples were heated at 92°C for 3 min before loading 10 µL of each serum sample onto. 5 µL molecular size standard (PPP) were used. Samples were separated using 1× Tris/Glycine/SDS buffer at 200 V for 40 min. The gels were activated for 45 s and scanned for 0.5-1 s using ChemiDoc Imaging system. To follow the *in vivo* elimination of imlifidase cleavage products, gel images from 15 individuals were evaluated using a visual scoring judging by the presence (+) or absence (-) of the F(ab')2 fragments over time.

### Results

Autologous or allogeneic cell therapies can be curative treatment options for patients with malignant (e.g. ALL, AML) and non-malignant diseases (e.g. immune deficiencies, Thalassemia). Safe and efficacious treatments need to overcome several hurdles, including the preconditioning of the host to eliminate malignant or defective hematogenic cells and to allow for the establishment of donor BMC. One of the challenges is to find treatment regimens that strikes a balance between sufficiently conditioning the host by eliminating pathogenic cells without compromising a swift reestablishment of the hematopoietic system by donor BMC. If this balance is not achieved, complications such as GvHD, graft failure (GF) and life-threatening infections may occur.

To achieve that balance, complex treatment regimens need to be adjusted for dosage and timing of e.g., irradiation and lymphodepleting drugs, like ATG or alemtuzumab. The IgG-based biologics rabbit ATG and anti-CD52 alemtuzumab bind and kill potentially both, donor- and host lymphocytes. On one hand, using high doses of these drugs leads to a desired reduction of graft-versus-host disease (GvHD), but at the cost of delayed immune reconstitution or even increased host engraftment failure, thereby enhancing the risk of severe infectious complications and overall survival (OS). Persisting levels of alemtuzumab or of ATG in the patient's blood (and bound to endothelial cells) creates yet another level of intricacy, delaying the immune recovery after HSCT which are associated with an increased risk of severe viral reactivations.

The dilemma of needing sufficiently high doses of IgG-based biologics during preconditioning, but low levels at the time of BMT can be solved using IgG-inactivating enzymes, such as imlifidase and EndoS. For this concept to work the biologics need to be cleavable by imlifidase. Fig. 3 shows that polyclonal rabbit ATG (ATG-G or Thymoglobulin), as well as, the human IgG1-based mAb alemtuzumab are efficiently cleaved in vitro by imlifidase, into their respective 2^{∗}Fc/2 and F(ab')2 fragments by imlifidase in buffer. Rabbit ATG and human IgG1-based alemtuzumab are thus examples of biologics that can be uncoupled from their Fc-effector functions and potentially be inactivated *in vivo* through imlifidase cleavage. In contrast, the horse polyclonal IgG thymoglobulin cannot be cleaved by imlifidase.

Even though the Fc-effector function of these antibodies was neutralized, it is still of interest if and at which dose their 2^{∗}Fc/2 and F(ab')2 fragments exert cytotoxic effect on target cells. The ability to kill K562 target cells was tested as a readout system to assess NK survival and functionality. NK cells were incubated with different concentrations of intact antibody (IgG) or their respective *in vitro*-cleaved 2^{∗}Fc/2 and F(ab')2 fragments.

The cytotoxic potency of rATG-F(ab')2 towards eNK cells was reduced compared to the equivalent concentrations of intact IgG (Fig.4). After incubation of NK cells with 100 µg/mL intact rATG only 20 % of the K562 target cells were lysed, demonstrating an impairment of the NK cells. In contrast, the equivalent dose of rATG F(ab')2 still allowed the NK cells to lyse most of their target tumor cells. This thus demonstrates that rATG F(ab')2 is less cytotoxic than intact rATG, even if it retains some of its cytotoxic activity at very high doses.

The protective effect of imlifidase cleavage of biologics is even more prominent when using the anti-CD52 targeting antibody alemtuzumab. A concentration of 2 µg/mL alemtuzumab efficiently inactivates the NK-effector cells, exhibiting an equivalent reduction of K562 target cell lysis at 100 µg/mL alemtuzumab. On the other hand, equivalent fragment doses of 100 µg/mL alemtuzumab, i.e. 75,3 µg/mL F(ab')2 or 24,7 µg/mL 2^{∗}Fc/2 fragments have no negative effect on NK cells. Neither 2^{∗}Fc/2 purified from imlifidase-cleaved rATG (Fig. 4c) nor from alemtuzumab had a blocking effects of NK cell activity.

ADCP is one of the effector mechanisms by which alemtuzumab can mediate the removal of CD52-positive target cells. Alemtuzumab was incubated with different concentrations of imlifidase to address the question which activity the different degrees of IgG digestion have on ADCP. The digests were run on SDS-PAGE to facilitate the visualization of the samples that were fully cleaved by imlifidase into F(ab')2 and 2^{∗}Fc/2 fragments, predominantly into scIgG, or intact alemtuzumab as positive control (Fig.5a). NuDUL1 target cells were incubated with either intact IgG, scIgG or fully imlifidase-cleaved F(ab')2 and 2^{∗}Fc/2 alemtuzumab fragments to test their ability to mediate ADCP by monocytic THP1 cells. At a concentration of 30 µg/mL both, single cleaved and intact alemtuzumab were able to mediate ADCP to the same degree (Fig.5b). A dose titration of the different alemtuzumab preparations shows that scIgG alemtuzumab completely loses its ability to opsonize CD52-positive NuDUL1 target cells at around 2 µg/mL, while intact alemtuzumab displays the same potency at 2 µg/mL and 30 µg/mL. Completely cleaved alemtuzumab into 2^{∗}Fc/2 and F(ab')2 by imlifidase fully loses its ability to mediate ADCP, even at 30 µg/mL.

After incubation with indicated doses of intact alemtuzumab or thymoglobulin, flow cytometry analysis of whole blood shows that both drugs efficiently reduce CD3+ positive T-cells already after 4 hours incubation (Fig. 6). In a next step, whole blood was preincubated with different doses of imlifidase for 6h before adding alemtuzumab (100 µg/mL) to the preconditioned blood and incubated for another 16h. Flow cytometry shows that imlifidase at 1.9 µg/mL is able to cleave endogenous IgG (Fig.7a) and able to block the cytotoxic effect of 100 µg/mL alemtuzumab on CD3-positive T-cells (Fig.7b).

In contrast, the horse IgG-based drug ATGAM is not cleavable by imlifidase (Fig. 3d), and its cytotoxic activity in whole blood is not reduced even at 30 µg/mL imlifidase (Fig. 8b), while the endogenous IgG pool is fully cleaved (Fig.8a).

The role of CDC in the early cytotoxic activity of alemtuzumab (30 µg/mL) and horse ATGAM (100 µg/mL) was shown by pre-incubating whole blood with the C5 inhibitor eculizumab. The complement cascade inhibition by eculizumab prevented most of the cytotoxicity by alemtuzumab and ATGAM. Single- and fully-cleaved alemtuzumab by imlifidase show the same degree of CDC blocking (Fig.9).

Imlifidase has not only the ability to cleave serum IgG *in vivo* but it also to cleaves therapeutic antibodies such as rabbit ATG and alemtuzumab into 2^{∗}Fc/2 and F(ab')2 fragments in the presence of serum matrix. The detachment of the Fc from IgG allows the F(ab')2 fragment not only to be eliminated faster due to its reduced molecular size but also due to the lack of FcRn-recycling, otherwise observed with intact IgG. Imlifidase-treated individuals exhibit a decline of F(ab')2 in serum when analyzed by SDS-PAGE, showing that full cleavage of intact IgG occurs within a couple of hours, as evidenced by the vanishing of the 150 kDa IgG band. Instead, a 100 kDa F(ab')2 and 25 kDa Fc/2 band is observed on SDS-PAGE. The intensity of the 100 kDa F(ab')2 band disappears to a great extend within 72 hours in most imlifidase-treated individuals (n=15), exemplified for one individual (ID-2) in Fig. 10a. These *in vivo* experiments show that imlifidase not only inactivates Fc-effector functions but also accelerates the decline of remaining F(ab')2 with potentially cytotoxic activity as observed for rATG.

### Discussion

Cell therapies such as hematopoietic stem cell transplantation (HSCT) can be curative treatment options for patients with malignant diseases, e.g., acute lymphoblastic leukemia (ALL), acute myeloid leukemia (AML), and for patients with non-malignant hematological diseases, e.g., immune deficiencies, Thalassemia and sickle cell disease. Preconditioning of the host is not only needed to create space for the donor HSC in the hematological niches, but also to suppress graft rejection by host immune cells, to avoid GvHD, and to eliminate the pathogenic host cells. Regimens including total body irradiation and administration of e.g. IgG-based biologics need to be fine-tuned to balance the desired with the negative effect from those treatments.

It has been shown that higher rATG doses correlate with lower BMTx rejection rates and lower incidence of acute and chronic GvHD. On the other hand, the same treatment also increases the risk of delayed immune recovery, i.e. NK and T-cell levels, rendering the patient more susceptible to life-threating infections. The recovery of the T-cell pool can exceed one year (Servais et al 2015). This problem can be solved by inactivating those IgG-based biologics prior to HSCT. The IgG-based biologics can be inactivated by imlifidase, which effectively hydrolyzes human and rabbit IgG in the lower hinge region creating one F(ab')2 fragment and one homodimeric 2^{∗}Fc/2-fragment. The resulting F(ab')2 fragment retains binding capacity to antigens (e.g. CD52) but their Fc-dependent effector mechanisms, such as complement-mediated cytotoxicity (CDC), antibody-dependent cell-mediated cytotoxicity (ADCC) and antibody-dependent cellular phagocytosis (ADCP) are eliminated. Since the mechanisms of action of rATG, Thymoglobulin and alemtuzumab are, to a large extent, Fc-mediated, imlifidase can be used as a tool to inactivate these drugs and limit cytotoxicity towards NK cells before HSCT. Furthermore, imlifidase is also of use in cases where some of the cytotoxic effector mechanism is mediated through the crosslinking of receptors, which would be achieved due to the accelerated elimination rate of F(ab')2 fragments compared to intact IgG.

Alternative methods of IgG removal, like plasmapheresis or plasma exchange, are inferior, since the drug antibodies i.e. rATG or alemtuzumab cannot be removed completely from the patient due to inevitable backflow from the tissue and interstitial fluids (Achini et al., 2020; Zhang et al., 2019). The same is true for already cell-bound antibodies. Imlifidase on the other hand, will inactivate Fc functions right away in the blood compartment as well as in interstitial fluid and even of already cell bound antibodies. The fast mode of action of imlifidase might also be useful in cases where no antidote is available for an accidental overdose of IgG-based drugs (van der Zwan et al., 2018), which need to be inactivated instantaneously. Imlifidase is also superior because it can be administered within minutes, whereas plasmapheresis or plasma exchange are more strenuous for patients.

### References

- Servais S, Menten-Dedoyart C, Beguin Y, Seidel L, Gothot A, Daulne C, Willems E, Delens L, Humblet-Baron S, Hannon M, Baron F. PLoS One. 2015 Jun 22;10(6):e0130026.
- Achini FR, Smiers F, Jan Zwaginga J, van Tol MJD, Jol-van der Zijde CM, Schilham MW, Lankester AC, Bredius RGM. Bone Marrow Transplant. 2020 Aug;55(8):1671-1673.
- Zhang P, Curley CI, Mudie K, Nakagaki M, Hill GR, Roberts JA, Tey SK. Bone Marrow Transplant. 2019 Dec;54(12):2110-2116.
- van der Zwan M, Baan CC, van Gelder T, Hesselink DA. Clin Pharmacokinet. 2018 Feb;57(2):191-207.

### Example 3 - dosing and timing intervals for imlifidase with alemtuzumab, ATG-G and Thymoglobulin

The following scheme for each of alemtuzumab, ATG-G and Thymoglobulin will be used in a clinical setting. Cell transplant takes place at day = 0.

| Step (a) | | | |
|---|---|---|---|
| Lymphodepleting agent | **Alemtuzumab** | **ATG-G** | **Thymoglobulin** |
| Total dose | Approx 1 mg/kg BW | Approx 30 mg/kg BW | Approx 10 mg/kg BW |
| Preferred administration protocol | 5 daily doses of 0.2 mg/kg BW (start on D -13) | 4 doses as follows: | 4 doses as follows: |
| | | 1× 1 mg/kg (d-12) | 1× 1 mg/kg (d-12) |
| | | 1× 9 mg/kg (d-11) | 1× 3 mg/kg (d-11) |
| | | 1× 10 mg/kg (d-10) | 1× 3 mg/kg (d-10) |
| | | 1× 10 mg/kg (d-9) | 1× 3 mg/kg (d-9) |
| Timing of last dose | D -9 | D -9 | D -9 |

| Step (b) | | | |
|---|---|---|---|
| Inactivating enzyme | imlifidase | imlifidase | imlifidase |
| Dose | Approx 0.25 mg/kg BW | Approx 0.25 mg/kg BW | Approx 0.25 mg/kg BW |
| Preferred administration protocol | Single dose (infusion) | Single dose (infusion) | Single dose (infusion) |
| Timing | D -2 | D -2 | D -2 |

## Claims

1. A conditioning regimen for the transplant of a cell to a subject, comprising:
a. administering to the subject an agent comprising IgG molecules, which agent reduces the numbers and/or down-modulates the activity of lymphocytes in the subject;
b. subsequently administering to the subject an enzyme which inactivates serum IgG molecules in the subject; and
c. subsequently administering the transplant to the subject.

2. The conditioning regimen of claim 1, wherein the transplant is of hematopoietic stem and progenitor cells (HSPC), which are optionally:
- autologous or allogenic; and/or
- genetically modified; and/or
- depleted of alpha/beta T-cells.

3. The conditioning regimen of claim 1 or 2, wherein the amount of the agent administered in step (a) is sufficient to substantially reduce the numbers and/or down-modulate the activity of lymphocytes in the subject, optionally wherein the agent is a rabbit antithymocyte globulin (rATG, e.g. ATG-G or Thymoglobulin) or an anti-CD52 monoclonal antibody (e.g. alemtuzumab), and optionally wherein the lymphocytes are T lymphocytes and/or B lymphocytes.

4. The condition regimen of any one of claims 1 to 3 wherein:
- The agent in step (a) is administered as one or more separate doses and the final dose of the agent is administered at least 1 day prior to step (c);
- The enzyme in step (b) is administered as one or more separate doses and the first dose of the enzyme is administered at least 1 day after the final dose of the agent in step (a), and optionally step (b) takes place on the same day as step (c).

5. The conditioning regimen of any one of claims 1 to 3 wherein the agent administered in step (a) is:
- an anti-CD52 monoclonal antibody (e.g. alemtuzumab) and the total dose administered is 0.5 - 250 mg/kg BW; or
- A rATG selected from ATG-G or Thymoglobulin, and the total dose administered is 5 - 80mg/kg BW for ATG-G or 0.5 - 15 mg/kg BW for Thymoglobulin.

6. The conditioning regimen of any one of the preceding claims, wherein the amount of the enzyme administered in step (b) is sufficient to inactivate all or substantially all IgG molecules present in the serum of the subject, optionally wherein the enzyme is an IgG cysteine protease or an IgG endoglycosidase.

7. The conditioning regimen of claim 6, wherein:
(i) the IgG cysteine protease is from a Streptococcus bacterium such as Streptococcus pyogenes, optionally wherein said enzyme is a IdeS, IdeZ or MAC2 polypeptide, or
(ii) the IgG endoglycosidase is from a Streptococcus bacterium, such as Streptococcus pyogenes, Streptococcus equi or Streptococcus zooepidemicus, or from Corynebacterium pseudotuberculosis, Enterococcus faecalis, or Elizabethkingia meningoseptica, optionally wherein said enzyme is a EndoS, CP40, EndoE, or EndoF2 polypeptide.

8. The conditioning regimen of claim 7, wherein:
- said IgG cysteine protease is a polypeptide comprising or consisting of a sequence that is at least 80% identical to SEQ ID NO: 2, 4, 5 such as at least 85%, 90%, 95%, 99% or 100% identical, or wherein said IgG cysteine protease comprises or consists of the sequence of any one of SEQ ID NOs: 6 to 25 and 55 to 69, 91 or 92, optionally wherein said sequence includes an additional methionine at the N terminus and/or a histidine tag at the C terminus; or
- said IgG endoglycosidase is a polypeptide comprising or consisting of a sequence that is at least 80% identical to SEQ ID NO: 90, such as at least 85%, 90%, 95%, 99% or 100% identical, optionally wherein said sequence includes an additional methionine at the N terminus and/or a histidine tag at the C terminus.

9. The conditioning regimen of any one of the preceding claims wherein in the enzyme is imlifidase and/or EndoS.

10. The conditioning regimen of any one of the preceding claims, which additionally comprises one or more of:
(a)a suitable interval before step c, typically 1 day, administering to the subject a non-lethal dose of irradiation and/or any other agent which depletes the subject's HSPC, for example Total Body Irradiation (TBI) at 4 Gy;
(b) administration of any other agent or regimen which modulates (e.g. reduces) the activity of the immune system , e.g., inhibitors of complement, inhibitors of cytokines, inhibitors of innate immune cells, inducers of tolerance.

11. A method for the prevention or treatment of immune rejection of a cell transplant, the method comprising administering a cell transplant to a patient in accordance with the conditioning regimen of any one of claims 1 to 10.

12. A method for the prevention or treatment of a disease or condition that is prevented or treated by cell transplant, the method comprising administering a cell transplant to a patient in accordance with the conditioning regime of any one of claims 1 to 10.

13. The method of claim 12, wherein the cell transplant is of HSPC and/or wherein the disease or condition is selected from:
- Hematological malignancies such as leukemias (for example Acute lymphoblastic leukemia (ALL), Acute myeloid leukemia (AML), Chronic lymphocytic leukemia (CLL), Chronic myelogenous leukemia (CML); lymphomas (for example Hodgkin's disease, Non-Hodgkin's lymphoma) and Myelomas (for example, Multiple myeloma (Kahler's disease));
- Hematologic diseases, such as such as phagocyte disorders (for example Myelodysplasia);
- Anemias (for example Paroxysmal nocturnal hemoglobinuria (PNH; severe aplasia), Aplastic anemia, Acquired pure red cell aplasia, Diamond-Blackfan anemia, Fanconi anemia); Hemoglobinopathies (for example Sickle cell disease, β thalassemia major (Cooley's anemia), Cytopenias (for example Amegakaryocytic thrombocytopenia); and Hemophagocytic syndromes (for example Hemophagocytic lymphohistiocytosis (HLH);
- Myeloproliferative disorders (for example Polycythemia vera, Essential thrombocytosis, Myelofibrosis);
- Solid tumor cancers, such as Neuroblastoma, Desmoplastic small round cell tumor, Ewing's sarcoma, Choriocarcinoma;
- Metabolic disorders such as amyloidosis (for example Amyloid light chain (AL) amyloidosis);
- Environmentally-induced diseases such as radiation poisoning;
- Viral diseases such as Human T-lymphotropic virus (HTLV) or Human Immunodeficiency Viruses (HIV);
- Autoimmune diseases, such as multiple sclerosis;
- Lysosomal storage disorders such as Lipidoses - disorders of lipid storage - (for example Neuronal ceroid lipofuscinoses, Infantile neuronal ceroid lipofuscinosis (INCL, Santavuori disease,), Jansky-Bielschowsky disease (late infantile neuronal ceroid lipofuscinosis)); Sphingolipidoses (for example Niemann-Pick disease, Gaucher disease); Leukodystrophies (for example Adrenoleukodystrophy, Metachromatic leukodystrophy, Krabbe disease (globoid cell leukodystrophy)); Mucopolysaccharidoses (for example Hurler syndrome (MPS I H, α-L-iduronidase deficiency), Scheie syndrome (MPS I S), Hurler-Scheie syndrome (MPS I H-S), Hunter syndrome (MPS II, iduronidase sulfate deficiency), Sanfilippo syndrome (MPS III), Morquio syndrome (MPS IV), Maroteaux-Lamy syndrome (MPS VI), Sly syndrome (MPS VII)); Glycoproteinoses (for example Mucolipidosis II (I-cell disease), Fucosidosis, Aspartylglucosaminuria, Alpha-mannosidosis; or Others (for example Wolman disease (acid lipase deficiency); and
- Immunodeficiencies, such as T-cell deficiencies (for example Ataxia-telangiectasia, DiGeorge syndrome); Combined T- and B-cell deficiencies (for example Severe combined immunodeficiency (SCID), all types); Wiskott-Aldrich syndrome; Phagocyte disorders (for example Kostmann syndrome, Shwachman-Diamond syndrome); Immune dysregulation diseases (for example Griscelli syndrome, type II); Innate immune deficiencies (for example NF-Kappa-B Essential Modulator (NEMO) deficiency.

14. A method for the treatment of alemtuzumab overdose, comprising administering to a patient in need thereof a therapeutically effective amount of an enzyme as defined in any one of claims 7 to 9.
